# EUROPEAN PATENT APPLICATION

(11) **EP 4 389 896 A1**
(43) Date of publication of application: **26.06.2024**
(21) Application number: 23822989.2
(22) Date of filing: 06.06.2023
(51) Int. Cl.: C12N 15/29, C12N 15/84, A01H 5/10, A01H 6/46, A01H 6/20

(54) **METHOD FOR INDUCING HAPLOIDY AND USE THEREOF IN PLANT BREEDING**

(30) Priority: 15.06.2022 CN 202210675450
(71) Applicant: China National Rice Research Institute, Hangzhou, Zhejiang 311401 (CN)
(72) Inventor: WANG, Kejian, Hangzhou, Zhejiang 311401 (CN); WANG, Chun, Hangzhou, Zhejiang 311401 (CN); WEI, Xin, Hangzhou, Zhejiang 311401 (CN)
(74) Representative: Ipside
(86) International application number: PCT/CN2023/098723
(87) International publication number: WO 2023/241420

(57) **Abstract**

The present invention relates to the field of biotechnology and plant breeding, and in particular relates to a method using plant cell proliferation regulator gene to induce haploid, and a use thereof in plant breeding. In the present invention, expression profiles of unfertilized egg cells of rice and rice embryos 5 days after fertilization are analyzed,and 2 genes are discovered to have relatively high *expression,OsCPROl* and *OsCPRO2.*The research shows that these two genes can be utilized to induce the production of haploid descendants.Specifically,a promoter specifically expressed in egg cells controls the gene to convert a plant,obtaining induced haploid plant material of a positive transgenic plant.Furthermore,the induced haploid plant material can be combined with a *MiMe* system to obtain apomixes material,and thereby generate clone seeds.

## Description

### Technical field

The present invention relates to the field of biotechnology and plant breeding, and in particular relates to a method using plant cell proliferation regulator gene to induce haploids, and a use thereof in plant breeding.

### Background technology

Compared with the traditional breeding methods, the haploid induction technology can obtain homozygous varieties within two generations, which can both shorten the breeding time and save unnecessary labor costs. There are two main source pathways for haploids, the first one comes from spontaneous formation in nature, and the second one comes from artificial methods to induce acquisition. Among them, the spontaneous formation of haploid ways in nature mainly include parthenogenesis, solitary male reproduction and sporophyte reproduction, but the occurrence frequency of these three pathways is very low (Zhang Zheng, 2007. Overview and thinking of research on haploid breeding. Shandong Agricultural Sciences, 5:122-125.DOI: 10.14083/j.issn.1001-4942.2007.05.025; Cui Ting, Li Yali, Qiao Linyi, Guo Huijuan, Dong Yanhui, Ren Yongkang, Tang Zhaohui, 2016. Wheat haploid breeding methods and its research progress. Shanxi Agricultural Sciences, 44 (1): 106-109.DOI: 10.3969/j.issn.1002-2481.2016.01.28), which cannot meet the needs of the breeding work. Breeders usually combine artificially induced haploids for assisted breeding.

There are many ways to induce haploids through artificial methods, and their induction rate is significantly higher than the frequency of natural occurrence. The methods for inducing parthenogenesis mainly include radiation induction, chemical induction, distant pollen stimulation induction, and induction using parthenogenesis induction lines(Xiang Zhiguo, Haiyan, Kang Minghui, Zhao Yong-ying, 2011. Haploid production way and its application in crop genetic breeding. Henan Agricultural Sciences, 40 (11): 17-21.DOI: 10.15933/j.cnki.1004-3268.2011.11.009). The methods for inducing solitary male reproduction mainly include: pollen in vitro culture, anther in vitro culture, and centromere mediated pathways (Li Ying, Wang Jia, Ji Lexiang, Li Hao, Ye Meixia, Guo Bin, Chen Zhong, An Xinmin, 2011, Research progress on plant haploid technology and its applications, Chinese Journal of Cell Biology, 33 (9): 1008-1014). The method of inducing sporophyte reproduction is called in vitro culture of female nuclei, which means in vitro culture of unpolluted organs such as ovaries or ovules to form new diploid plants.Haploid induction technology has been applied to breeding efforts in many crops, for example, rice, wheat, corn, cotton, barley, tobacco and rape.Its development is the most mature in maize, and has created a series of high frequency induction lines: WS14, EMK, Nongda Gaoyou 1 (NG1) and Ji Gaoyou 3 (JG3), among them, the induction rate of NG1 was 4% to 6%, the induction of JG3 was 10%. The application of these induced lines has greatly accelerated the process of maize breeding (Cai Zhuo, Xu Guoliang, Liu Xianghui, Dong Yalin, Dai Yuxian, Li Shuhua, 2007. The breeding of maize high frequency single reproductive induction line 3, Maize Science, 15(1):1-4.DOI: 10.13597/j.cnki.maize.science.2007.01.001).

In 2015, Conner et al. (Conner J A, Mookkan M, Huoh, Chae K, Ozias-Akins P, 2015. A parthenogenesis gene of apomict origin elicits embryo formation from unfertilized eggs in a sexual plant.PNAS, 112(36): 11205-11210.DOI: 10.1073/pnas.1505856112.) found that the *PsASGR-BBML* gene of African wolf tail grass was expressed in the ovary, developing seeds, roots and anthers before fertilization, and that the *PsASGR-BBML* transgene could promote the induction of pearl millet haploid embryos in sexual reproductive species. In 2017, Conner et al. (Conner J A, Podio M, Ozias-Akins P, 2017. Haploid embryo production in rice and maize induced by PsASGR-BBML transgenes.Plant Reprod, 30(1): 41-52.DOI: 10.1007/s00497-017-0298-x.) subsequent studies found that PsASGR-BBML transgenes induced the production of haploid embryos in two major monocot crops, maize and rice. In 2019, Khanday et al. (Khanday I, Skinner D, Yang B, Mercier R, Sundaresan V, 2019. A male-expressed rice embryogenic trigger redirected for asexual propagation through seeds. Nature, 565 (7737): 91-95.DOI: 10.1038/s41586-018-0785-8.) found that the expression of *BBM1* in rice started from male gametes, activated the expression of the female genome, and then acted on the development process of the whole embryo. Ectopic expression of *OsBBM1* in egg cells using *AtDD45* promoter can induce parthenogenesis, with an induction rate of 5% to 10%, the induction rate of theT₂transgenic material can reach 29%. The ectopic expression of *BBM1* in eggs was combined with the *MiMe* (*osdl* / *rec8* / *spoll-1*) system, that is, the process of replacing meiosis in primitive germ cells with mitosis during parthenogenesis, and the whole genome was obtained.

In 2017, Kelliher, et al. (Kelliher T, Starr D, Richbourg L, Chintamanani S, Delzer B, Nuccio M L, Green J, Chen Z Y, Mccuiston J, Wang W L, Liebler T, Bullock P, Martin B, 2017. MATRILINEAL, a sperm-specific phospholipase, triggers maize haploid Induction. Nature, 542 (7639): 105-109.DOI: 10.1038/nature20827.) found that the *MTL* gene is expressed in the cytoplasm of sperm, and the frameshift mutation could induce the production of haploid with an induction rate of 6.7%. In 2018, Yao et al. (YaoL, Zhang Y, Liu C X, Liu Y B, Wang Y L, Liang D W, Liu J T, Sahoo G, Kelliher T, 2018. OsMATL mutation induces haploid seed formation in indica rice.Nature Plants,4(8): 530-533.DOI: 10.1038/s41477-018-0193-y.) in order to develop a haploid inducible gene in rice, *OsMTL* homologous to *ZmMTL* was found, and it was found that knockout of this gene can induce the production of haploid in rice, with an induction rate of 2%~6%, which proves that *MTL* is also involved in mediating the formation of haploid in rice, and can be used to construct haploid inducible lines in rice. In 2019, Wang, et al. (Wang C, Liu Q, Shen Y, Hua Y F, Wang J J, Lin J R, Wu M G, Sun T T, Cheng Z K, Mercier R, Wang K J, 2019. Clonal seeds from hybrid rice by simultaneous genome engineering of meiosis and fertilization genes. Nature Biotechnology, 37 (3): 283-286.DOI: 10.1038/s41587-018-0003-0.) knockout out the *OsMTL* gene in CY84 of hybrid rice to obtain *mtl* haploid induction material of hybrid rice background with an induction rate of about 5% and seed set rate of about 10%. In addition, Fix (Fixation of hybrid) material was obtained by simultaneously knocking out four endogenous genes of *REC8, PAIR1*, *OSD1* and *MTL,* completing the fixation of heterosis.

Although there are many methods to induce haploid production, there are few genes that can achieve haploid induction only by single gene regulation, and even fewer genes that can be applied to produce clonal seeds in rice apomicis. Currently, there are only two genes, *OsBBM1* and *OsMTL.*

### Invention content

Through the analysis of expression profiles of rice unfertilized egg cells and embryos 5 days after fertilization, two genes with high expression were found, and two new genes with haploid induction ability were found in rice, named *OsCPRO1 (CELL PROLIFERATION!*) and *OsCPRO2 (CELL PROLIFERATION2*) respectively, the gene symbol of *OsCPRO1* was *LOC_Os03g47740,* and the gene symbol of *OsCPRO2* was *LOC_Os12g43950.* Moreover, these two haploid-inducible genes can be applied to apomixis in rice, realizing the fixation of heterosis and the formation of clonal seeds.

Two new genes with haploid induction ability, *OsCPRO1 (LOC_Os03g47740)* and *OsCPRO2* (*LOC_Os12g43950*), were found in rice. This haploid induction material has high seed setting rate and can be applied to apomixis reproduction in rice.

Therefore, the invention provides a protein with haploid induction ability, including the POX superfamily domain and Homeobox domain, specifically, POX, the superfamily domain containing BELL domain and SKY domain: SKYLKAAQELLDEVVSV; preferably it is a *OsCPRO1* or OsCPRO2 protein, amino acid sequences are shown in SEQ ID NO: 3 and SEQ ID NO: 6, respectively, or their orthologous genes derived from the following species, optimally greater than 90%, 95%, more than 98% or 99%:rice *(Oryza sativa),* wart-grain rice *(Oryza meyeriana varY,* maize(*Zea mays*), wheat *(Triticum aestivum),* durum wheat *(Triticum turgidum subsp),* wild emmer wheat(*Triticum dicoccoides),* urartu wheat *(Triticum urartu),* barley *(Hordeum vulgare),* section wheat (*Aegilops tauschii),* rye *(Secale cereale),* oats (*Avena sativa),* buckwheat *(Fagopyrum esculentum),* highland barley *(Hordeum vulgare),* semen coicis (*Coixlacryma-jobi*), foxtail millet*(Setariaitalica),* fonio millet *(Digitariaexilis),* sorghum *(Sorghum bicolor),* proso *millet(Panicum miliaceum),* wild rice *(Zizania latifolia),* biotic wild rice *(Zizania palustris), coracana(Eleusine coracana),* sweet potato *(Dioscorea esculenta),* cassava *(Manihot esculenta),* potato *(Solanum tuberosum),* yam*(Dioscoreapolystachya),* groundnut *(Arachis hypogaea),* cranes peanut *(Arachis duranensis),* soybean *(Glycine max),* wild soybean *(Glycine soja),* adzuki bean *(Vigna angularis),* red bean *(Vigna umbellata),* lentil bean *(Lens culinaris),* mung bean *(Vigna radiata),* cowpea *(Vigna unguiculata),* kidney bean *(Phaseolus vulgaris),* broad bean *(Vicia faba),* pea *(Pisum sativum),* jack bean *(Canavalia gladiata),*pigeonpea *(Cajanus cajan),* white lupine *(Lupinus albus),* lather bean*(Mucuna pruriens),* narrow-leafed lupine *(Lupinus angustifolius),* hairy vine bean *(Calopogoniummucunoides),* chickpea *(Cicer arietinum),* sesame *(Sesamum indicum),* flax *(Linumusitatissimum),* rapeseed *(Brassica napus),* sunflower *(Helianthus annuus),* castor bean *(Ricinus communis),* red flower *(Carthamus tinctorius),* beet *(Beta vulgaris),* sugarcane *(Saccharum officinarum),* cotton *(Gossypium spp), marijuana(Cannabis sativa),* jiangnan rolling cypress *(Selaginella moellendorffii),* lemon eucalyptus *(Corymbia citriodora),* water green tree *(Tetracentronsinense),* mesquite tree *(Prosopis alba),* taxus chinensis *(Taxus chinensis),* olive *(Olea europaea),* African acacia*(Abrusprecatorius),* switchgrass *(Panicum virgatum),* red-vein maiguo *(Rhamnellarubrinervis),* American hickory *(Carya illinoinensis),* tea *(Camellia sinensis),* tobacco *(Nicotiana tabacum),* elephant grass *(Pennisetum purpureum),* hard-straight ryegrass *(Lolium rigidum),* nandi *(Miscanthuslutarioriparius),* canweed *(Setariaviridis),* cockweed *(Pennisetum alopecuroides),* Sudan grass *(Sorghum sudanense),* Tang pine grass *(Thalictrum thalictroides),* curved leaf thrush *(Eragrostiscurvula),* Brachypodium *(Brachypodiumdistachyon),* alfalfa *(LotuscorniculatusL),* Arabidopsis *(Arabidopsis thaliana),*Physcomitrella*(Physcomitrium patens),* hornmoss *(Ceratodonpurpureus),* cabbage *(Brassica oleracea),* mustard *(Brassica juncea),* Chinesegreen cabbage *(Brassica rapa),* cauliflower *(Brassica oleracea),* lettuce *(Lactuca sativa),* spinach *(Spinacia oleracea), radish(Raphanus sativus),* Chinesecabbage *(Brassica rapa),* zucchini *(Cucurbita pepo),* hemsley *(Apium graveolens),* leek *(Allium tuberosum),* carrot *(Daucus carota),* eggplant*(Solanum melongena),* tomato *(Lycopersicon esculentum),* cucumber *(Cucumis sativus),* wax gourd *(Benincasahispida),* pumpkin *(Cucurbita moschata),* loofah *(Luffa cylindrica),* vegetable melon *(Cucumis melo),* lotus vegetables *(Potentilla anserina),*day lily *(Hemerocallis citrina),* stem lettuce *(Lactuca sativa),* asparagus *(Asparagus officinalis),* chili pepper *(Capsicum annuum),* garlic *(Allium sativum),* spring onion *(Allium fistulosum),* Jerusalem artichoke *(Helianthus tuberosus),* Coriander *(Coriandrum sativum),* watermelon *(Citrullus lanatus),* pear *(Pyrus spp),* peach *(Prunus persica),* apricot *(Armeniaca vulgaris),* lee *(Prunus salicina),* date *(Ziziphus jujuba),* green plum *(Vaticamangachapoi),* apple *(Malus pumila),* sand fruit *(Malus asiatica),* cherry *(Cerasus spp),* walnut *(Juglans regia),* strawberry *(Fragaria ananassa),* grape *(Vitis vinifera), pineapple* pear *(Ananas comosus),* coptis chinensis *(Coptis chinensis),* Chinese milk vetch *(Astragalus sinicus),* ginseng *(Panax ginseng),* Angelica sinensis *(Angelica sinensis),* honeysuckle *(Lonicera japonica),* Ai *(Artemisia argyi),* peppermint *(Mentha canadensis),* orchid *(Cymbidium ssp),* lily *(Lilium brownii),* tulip *(Tulipa gesneriana).*

Correspondingly, the invention provides the proteinencoding genes, which is either *OsCPRO1* or *OsCPRO2,* where the gene symbol of *OsCPRO1* is *LOC_Os03g47740,* the gene symbol of *OsCPRO2* is *LOC_Os12g43950*; or BEL1-like homeodomain protein 6 derived from maize, Sequence ID: PWZ21223.1; or BEL1-like homeodomain protein 7 derived from wheat, Sequence ID: XP_044365192.1; or BEL1-like homeodomain protein 1 derived from soybean, Sequence ID: XP_003543416.1; or BEL1-like homeodomain protein 1 from groundpeanut, Sequence ID: XP_016170518.1; or at least 90%, 95% with the above genes, more preferably more than 98%, or 99% of the homologous genes derived from the same species, and still has the haploid induction ability.

More preferably, the nucleotide sequence of *OsCPRO1* is as shown in SEQ ID NO: 1, or more than 90%, 95%, 98%, and still has haploid induction ability; the nucleotide sequence of *OsCPRO2,* as shown in SEQ ID NO: 4, is more preferably more than 98% and 99%, and still has haploid induction ability.

In addition, the CDS nucleotide sequence of *OsCPRO1* is as shown in SEQ ID NO: 2, or from rice still has haploid induction ability with more than 98% or 99% identity, and still has haploid induction ability; the CDS nucleotide sequence of *OsCPRO2* is as shown in SEQID NO: 5, or from rice still has more than 98% or 99% identity.

The invention provides an expression cassette, recombinant vector, recombinant cell or host cell containing the gene.

The invention also provides for the protein, or the application of the genes in inducing plant haploids.

The present invention further provides a method for inducing plant haploids using the gene, comprising the steps of transforming a plant with a recombinant expression vector containing the gene to obtain a positive transgenic plant; wherein the gene is controlled by the promoter of specific expression of the egg cell or, preferably, the plant includes a monocot and dicotyledon;

Preferably, the promoter specifically expressed in egg cells is *AtDD45* egg cells specific promoter, and more preferably, with the nucleotide sequence as described in SEQ ID NO: 7; the import is Agrobacterium-mediated or gene gun or gene editing.

More specifically, include the following steps: survey of wild-type plants and field phenotypic traits of T₀transgenic-positive plants; Seeds of the T₀transgenic positive material were replaced and germinated to obtain T₁transtransgenic plants; using Indel markers or flow cell ploidy detection for T₁generation of transgenic positive material for haploid detection to obtain haploid material. Further, including combining haploid induction material with the *MiMe* system to obtain apomictic material.

The haploid-induced genes found in the existing technologies are only *OsBBMl* and *OsMTL,* which have been applied to the artificial creation of apomixis. Two new genes (*OsCPRO1* and *OsCPRO2*) with haploid induction ability found by the present invention can also be applied to produce clonal seeds and have high application value.

### Attached figure description

Figure1.Plant type of the T₀generation *OsCPRO* series of haploid-inducing materials. Where, CY84: control; *OsCPRO-ee:* haploid induction material; percentage: seed set; scale bar =10 cm.
Figure2.Head pattern of the T₀generation *OsCPRO* series of haploid-inducing material. Where, CY84: control; *OsCPRO-ee*: haploid induction material; percentage: seed set; scale bar =5 cm.
Figure 3.Genotyping of the Indel markers used for haploid detection.
Figure4.Detection of Indel markers in T₁generation haploid and double haploid material. Where, 16A, C84 and CY84: parental control; numbers for haploid and double haploid material.
Figure5.Flow cytometric ploidy detection ofT₁ generation haploid and double haploid materials. Where, A: haploid and diploid control; B: haploid and double haploid material.
Figure 6. Comparison of haploid material with wild-type CY84, where A: comparison of plant type, scale bar =10 cm; B: comparison of spike type, scale bar =5 cm.
Figure 7.Trait comparison of double-haploid material with wild-type CY84. A: comparison of plant type, scale =10 cm; B: comparison of ear type, scale =5 cm.
Figure 8.Trait comparison between haploid material and Col-0 ecotype wild Arabidopsis. A: wild-type, B: haploid, scale bar =1 cm.
Figure 9.Overview of the technical route of the present invention.

### Specific implementation methods

The present invention is further explained in combination with the embodiments. These embodiments are used only to illustrate the invention and not to limit the scope of the invention.

### Example 1: Discovery and characteristics of OsCPRO1 and OsCPRO2

Through the analysis of expression profiles of rice unfertilized egg cells and embryos 5 days after fertilization, two genes with high expression were found, and two new genes with haploid induction ability were found in rice, named *OsCPRO1* (*CELL PROLIFERATION1*) and *OsCPRO2* (*CELL PROLIFERATION2*) respectively, the gene symbol of *OsCPRO1* was *LOC_Os03g47740,* and the gene symbol of *OsCPRO2* was *LOC_Os12g43950.*

### 1. OsCPRO1 (LOC_Os03g47740)

(1) Gene sequence of *OsCPRO1* (*LOC_Os03g47740*), as shown in SEQ ID NO: 1.
(2) The *OsCPRO1* (*LOC_Os03g47740*) CDS sequence, as shown in SEQ ID NO: 2.
(3) The amino acid sequence of *OsCPRO1* (*LOC_Os03g47740*), as shown in SEQ ID NO: 3.
(4) *OsCPRO1* (L*OC_Os03g47740*) important domains:
   ① POX superfamily domains (pfam07526): a family of protein domains associated with nuclear function. Contains the -BELL domain: LQNKMAKLMAMLDEVDRKYKHYYHQMQIVVSSFDMVAGSGAAKPYTAVALQTISKHF RCLKDAINDQINVIRKKLGEEESSSGKEGKLTRLRYIDQQLRQQRAFQQYGLLQ and the SKY domain: SKYLKAAQELLDEVVSV.
   ②Homeobox domain (pfam05920): this is a homeodomain transcription factor KN domain conserved from fungi to humans and plants. They were originally identified in eukaryotes as the TALE homeobox genes (including the KNOX and MEIS genes).

### 2. OsCPRO2 (LOC_Os12g43950)

(1) Gene sequence of *OsCPRO2* (*LOC_Os12g43950*), as shown in SEQ ID NO: 4.
(2) The *OsCPRO2* (*LOC_Os12g43950*) CDS sequence, as shown in SEQ ID NO: 5.
(3) The amino acid sequence of *OsCPRO2* (*LOC_Os12g43950*), as shown in SEQ ID NO: 6.
(4) *OsCPRO2* (*LOC_*O*s12g43950)* important domains:
   ① POX superfamily domains (pfam07526): a family of protein domains associated with nuclear function. Contains the BELL domain: LQNKMAKLMAMLDEVDRKYKHYYHQMQTV V SSFDV VAGPGSAKPYTAVALQTISRHF RCLKDAINDQINVIRKKLGEEENSSGKEGKLTRLRYIDQQLRQQRAFQQYGMIP and the SKY domain: SRYLKAAQELLDEVVSV.
   ②omeobox domain (pfam05920): this is a homeodomain transcription factor KN domain conserved from fungi to humans and plants. They were originally identified in eukaryotes as the TALE homeobox genes (including the KNOX and MEIS genes).

### 3.The species where the OsCPRO1 and OsCPRO2 homologs are located

Homology alignment of amino acid sequences was performed using the NCBI database, including maize, wheat, soybean and groundnut. The results of amino acid sequence alignment of *OsCPRO1* and OsCPRO2 homologous are as follows:
Maize [*Zea mays*], BEL1-like homeodomain protein 6, Sequence ID: PWZ21223.1Length: 643Number of Matches: 1, Range 1:1 to 634.

| Score | | Expect | Method | Identities | Positives | Gaps |
|---|---|---|---|---|---|---|
| 955 bits(2469) | | 0.0 | Compositional matrix adjust. | 499/642(78%) | 559/642(87%) | 15/642(2%) |
| Query | 1 | MATYYSSPGNERDSQAMYPADSGNSSYPVPSAIGNMLYPGNGSSGPYTEFSGIIQHQQNF | | | | 60 |
| Sbjct | 1 | MATYYSSPDSERDSQTMYSTESGNASYPVPSALGNFLYLNSASSGPYTEFNGIVQSQQNF | | | | 60 |
| Query | 61 | MELPGHPTAISQDSSSRE-PNMVASYMDQRSFGPAKDMRNEMLMHLMDGAHNAGADLIHN | | | | 119 |
| Sbjct | 61 | MELTGHPSAISHDSSSNEATNIGTSLTEQRSFGPLKDMRNEMLMHLMDGAHSSGSDLIHN | | | | 120 |
| Query | 120 | DTHSSAQIEFGLLNNHNSMSVAPAPGQGLSLSLNTHILAPSYPYWSAKTELLTPHSYHGD | | | | 179 |
| Sbjct | 121 | DDHSTAQLEFGMLNNHNSTSLPSASGQGLSLSLNTHILAPSYPYWSAKQDLLTPNSYQGD | | | | 180 |
| Query | 180 | DNRMKNMQSEASQAIRNSKYLKAAQELLDEVVSVWKS1KQKAQKDQAEAGKSDNKEAEGG | | | | 239 |
| Sbjct | 181 | DNRMKNMQSEASQAIRNSKYLKAAQELLDEIVSVWKSVKQKTDKGPSEAGKSDGKETDGG | | | | 240 |
| Query | 240 | SKGEGVSSNPQESTANAAPEISAAEKQELQNKMAKLMAMLDEVDRKYKHYYHQMQIVVSS | | | | 299 |
| Sbjct | 241 | TKSEGVSFDPQESGANTAAELSTAEKQELQNKMAKLMAMLDEVDRKYKHYYHRMQLVMSS | | | | 300 |
| Query | 300 | FDMVAGSGAAKPYTAVALQTISKHFRCLKDAINDQINVIRKKLGEEESSSGKEGKLTRLR | | | | 359 |
| Sbjct | 301 | FDMVAGSGAAKPYTAVALQTISRHFRCLKDAINDQISVIRKKLGEDDDASGKEGKLIRLR | | | | 360 |
| Query | 360 | YIDQQLRQQRAFQQYGLLQQNAWRPQRGLPENSVSILRAWLFEHFLHPYPKDSEKLMLAR | | | | 419 |
| Sbjct | 361 | YIDQQIRQQRAFQQYGMLQQNAWRPQRGLPENSVSILRAWLFEHFLHPYPKDSEKLMLSR | | | | 420 |
| Query | 420 | QTGLTRSQISNWFINARVRLWKPMIEDMYKEEI--GEADLDSNSSSDNVPRSKDKIATSE | | | | 477 |
| Sbjct | 421 | QTGLTRSQISNWFINARVRLWKPMIEDMYKEEIGEGEAELDSNSSSDNVQRNKDKPPSSE | | | | 480 |
| Query | 478 | DKEDLKSSMSQTYQPSQLGESKANI--GMMSLGGAPA--GFHNEGNQDDSFMNLMLKDQRPG | | | | 535 |
| Sbjct | 481 | EKEDHKTSTSQVCQTSQLGESKSNIGGLMSFSGAPAGGFHNDVNPDDSFMSLMLKAQRPG | | | | 540 |
| Query | 536 | EAEGS-LLHDAVAHHSDENARFMAYHLSGLGRYGNSNVSLTLGLQHPDNRLSVQNTHQPG | | | | 594 |
| Sbjct | 541 | ETDGSGLLHDAVAHHSDESARFMAYHLTEFGRYGNNNVSLTLGLQHAENT -------QPG | | | | 593 |
| Query | 595 | FAGA-GEEIYNSTASEGVAAASSSDYESTNQIDQRQRSSCRI | | | | 635 |
| Sbjct | 594 | FPGVRDQDIYNSTAPLNV-TSTSSEYDSASQIDQQQRQRFEV | 634 | | | |

Wheat [*Triticum aestivum*], BEL1-like homeodomain protein 7, Sequence ID: XP_044365192. 1Length: 638Number of Matches: 1, Range 1:1 to 626.

| Score | | Expect | Method | Identities | Positives | Gaps |
|---|---|---|---|---|---|---|
| 944 bits(2441) | | 0.0 | Compositional matrix adjust. | 503/636(79%) | 548/636(86%) | 16/636(2%) |
| Query | 1 | MATYYSSPGNERDSQAMYPADSGNSSYPVPSAIGNMLYPGNGSSGPYTEFSGIIQHQQNF | | | | 60 |
| Sbjct | 1 | MSNYYSSPGDERDPQTMYSPDTGNASYPVPSALGNLLYSNNASSGPYTEFSGIIQPQQNF | | | | 60 |
| Query | 61 | MELPGHPTAISQDSSSREP-NMVASYMDQRSFGPAKDMRNEMLMHLMDGAHNAGA--DLI | | | | 117 |
| Sbjct | 61 | MELHGHP---SEHSSSREPPNMVTSLTEQSSFAPVKDMRNEMLMHFMDGAQSGGGGGDLI | | | | 117 |
| Query | 118 | HNDTHSSAQIEFGLLNNHNSMSVAPAPGQGLSLSLNTHILAPSYPYWSAKTELLTPHSYH | | | | 177 |
| Sbjct | 118 | HNDAHSSAQLDFGLLNNPSSASVPSAPGQGLSLSLNTHILAPSYPYWSPKPDLLTTQSYQ | | | | 177 |
| Query | 178 | GDDNRMKNMQSEASQAIRNSKYLKAAQELLDEVVSVWKSIKQKAQKDQAEAGKSDNKEAE | | | | 237 |
| Sbjct | 178 | GDENGMKNMQSEASRAIRNSKYLKAAQELLDEIVSVWKSIKQNAQKEKAEAGKMDGKDAD | | | | 237 |
| Query | 238 | GGSKGEGVSSNPQESTANAAPEISAAEKQELQNKMAKLMAMLDEVDRKYKHYYHQMQIVV | | | | 297 |
| Sbjct | 238 | EVLKSEGVSSNPQESTANAEAEISAAEKQELQNKMAKLLAMLDEVDRKYKHYFHQMQIVV | | | | 297 |
| Query | 298 | SSFDMVAGSGAAKPYTAVALQTISKHFRCLKDAINDQINVIRKKLGEEESSSGKEGKLTR | | | | 357 |
| Sbjct | 298 | SSFDMIAGSGAAKPYTAVALQTISRHFRCLKDAINDQVNVIRKKLGEEDSSSGREGKLTR | | | | 357 |
| Query | 358 | LRYIDQQLRQQRAFQQYGLLQQNAWRPQRGLPENSVSILRAWLFEHFLHPYPKDSEKLML | | | | 417 |
| Sbjct | 358 | LRYIDQQLRQQRAFQQYGMLQQNAWRPQRGLPENSVSILRAWLFEHFLHPYPKDSEKLML | | | | 417 |
| Query | 418 | ARQTGLTRSQISNWINARVRLWKPMIEDMYKEEIGEADLDSNSSSDNVPRSKDKIATSE | | | | 477 |
| Sbjct | 418 | ARQTGLTRSQISNWFINARVRLWKPMIEDMYKEETGEAELDSNSSSDNLPRSKDKMASCE | | | | 477 |
| Query | 478 | DKEDLKSSMSQTYQPSQLGESKANIGMMSLGGAPAGFHNEGNQDDSFMNLMLKDQRPGEA | | | | 537 |
| Sbjct | 478 | DKEDLKCSMSQG-QAYQTSEFKANMEMAGLTGAPSSFHNEANSDDGFMNLLLKDQRPGEA | | | | 536 |
| Query | 538 | EGSLLHDAVAHHSDENARFMAYHLSGLGRYGNSNVSLTLGLQHPDNRLSVQNIHQPGFAG | | | | 597 |
| Sbjct | 537 | DGSLL-----HGDESARFMAYHLAELGGYQNSNVSLTLGLQHTENSLSAPNAHRPGFTA | | | | 590 |
| Query | 598 | AGEE-IYNSTASL--GVAAASSSDYESTNQIDQRQR | | | | 630 |
| Sbjct | 591 | AGEEDIYNTTAANPGGGAAVASSDYESTNQLDQRQQ | | | | 626 |

Soybean [*Glycine max*], BELl-like homeodomain protein 1, Sequence ID: XP_003543416.1Length: 702Number of Matches: 1, Range 1:166 to 489.

| Score | | Expect | Method | Identities | Positives | Gaps |
|---|---|---|---|---|---|---|
| 273 bits(697) | | 1e-74 | Compositional matrix adjust. | 162/359(45%) | 218/359(60%) | 46/359(12%) |
| Query | 413 | MDSGGRKHLASSSYSGPSGTAGSSNHISASKFLRSAQAILNEVCRVTPLKRPPKSVRSSD | | | | 472 |
| Sbjct | 166 | LDVAGQGHVAGIGNSPMSASIGVSGVIMGSKYLKAAQELLDEVVNVC------KGIY--- | | | | 216 |
| Query | 473 | QQHWSMAGGSSTSVDANLTYNGREERSGMLAGEVDSARDPASFvttsslvtvsQVPLESE | | | | 532 |
| Sbjct | 217 | ---------------------KEEK---FSEKVKANRESTNSGAAGDGGDGSSGGGENS | | | | 251 |
| Query | 533 | MIQGLAEAARCESRDDLELKKQKLSLMLDEVEARYRRYCDHLQLVITGFNSQAGPNTATP | | | | 592 |
| Sbjct | 252 | AGKQVVELSTAQ-RQELQMKKSKLVTMLDEVEQRYRQYHHQMQIVVSSFEQAAGYGAAKS | | | | 310 |
| Query | 593 | YTILALQAMSRHFRCLKDAIGSQLRIVKRTLGEDDRTG-QGETSRLRYVDqqirqqralq | | | | 651 |
| Sbjct | 311 | YTALALKTISKQFRCLKDAISAQIKATSKTLGEDDCLGVKVEGSRLRFVDHHLRQQRALQ | | | | 370 |
| Query | 652 | q1GMLQQHAWRPQRGLPERAVSVLRAWLFEHFLHPYPKDVDKLSLAKQTGLTRSQVSNWF | | | | 711 |
| Sbjct | 371 | QLGMIQPNAWRPQRGLPERAVSILRAWLFEHFLHPYPKDSDKVMLAKQTGLARSQVSNWF | | | | 430 |
| Query | 712 | INARVRLWKPMVEEMYVEEQKEY---------------------SEDHSTALAQSERMARDQVETENNTY | | | | 760 |
| Sbjct | 431 | INARVRLWKPMVEEMYLEEIKEHEQGNGSENTKSKESSKELASTANVALDHLQSKHESF | | | | 489 |

[*Arachis ipaensis*], BEL1-like homeodomain protein 1, Sequence ID: XP_016170518.1Length: 733Number of Matches: 1 Range 1:278 to 488.

| Score | | Expect | Method | Identities | Positives | Gaps |
|---|---|---|---|---|---|---|
| 269 bits(687) | | 4e-73 | Compositional matrix adjust. | 139/211(66%) | 162/211(76%) | 1/211(0%) |
| Query | 546 | RDDLELKKQKLSLMLDEVEARYRRYCDHLQLVITGFNSQAGPNTATPYTILALQAMSRHF | | | | 605 |
| Sbjct | 278 | RQELQMKKSKLVCMLDEVEQRYRQYHHQMQVVISSFEQAAGFGAAKSYTSLALKTISKQF | | | | 337 |
| Query | 606 | RCLKDAIGSQLRIVKRTLGEDDRTG-QGETSRLRYVDqqirqqralqq1GMLQQHAWRPQ | | | | 664 |
| Sbjct | 338 | RCLKDAISSQIRATSKTLGEDDCLGAKVEGSRLRYVDHHLRQQRALQQLGMIQPNAWRPQ | | | | 397 |
| Query | 665 | RGLPERAVSVLRAWLFEHFLHPYPKDVDKLSLAKQTGLTRSQVSNWFINARVRLWKPMVE | | | | 724 |
| Sbjct | 398 | RGLPERAVSILRAWLFEHFLHPYPKDSDKVMLAKQTGLTRSQVSNWFINARVRLWKPMVE | | | | 457 |
| Query | 725 | EMYVEEQKEYSEDHSTALAQSERMARDQVEI | | | | 755 |
| Sbjct | 458 | EMYMEEVKEQEINNNNNNNGSERSKESSKEL | | | | 488 |

After verification, homologous genes in species include but are not limited to: rice *(Oryza sativa),* wart-grain rice *(Oryza meyeriana var),* maize(*Zea mays),* wheat *(Triticum aestivum),* durum wheat *(Triticum turgidum subsp),* wild emmer *wheat(Triticum dicoccoides),* urartu wheat *(Triticum urartu),* barley *(Hordeum vulgare),* section wheat (*Aegilops tauschii),* rye *(Secale cereale),* oats (*Avena sativa),* buckwheat *(Fagopyrum esculentum),* highland barley *(Hordeum vulgare),* semen coicis (*Coixlacryma-jobi*), foxtail millet*(Setariaitalica),* fonio millet *(Digitariaexilis),* sorghum *(Sorghum bicolor),* proso *millet(Panicum miliaceum),* wild rice *(Zizania latifolia),* biotic wild rice *(Zizania palustris), coracana(Eleusine coracana),* sweet potato *(Dioscorea esculenta),* cassava *(Manihot esculenta),* potato *(Solanum tuberosum),* yam*(Dioscoreapolystachya),* groundnut *(Arachis hypogaea),* cranes peanut *(Arachis duranensis),* soybean *(Glycine max),* wild soybean *(Glycine soja),* adzuki bean *(Vigna angularis),* red bean *(Vigna umbellata),* lentil bean *(Lens culinaris),* mung bean *(Vigna radiata),* cowpea *(Vigna unguiculata),* kidney bean *(Phaseolus vulgaris),* broad bean *(Vicia faba),* pea *(Pisum sativum),* jack bean *(Canavalia gladiata),*pigeonpea *(Cajanus cajan),* white lupine *(Lupinus albus),* lather bean*(Mucuna pruriens),* narrow-leafed lupine *(Lupinus angustifolius),* hairy vine bean *(Calopogoniummucunoides),* chickpea *(Cicer arietinum),* sesame *(Sesamum indicum),* flax *(Linumusitatissimum),* rapeseed *(Brassica napus),* sunflower *(Helianthus annuus),* castor bean *(Ricinus communis),* red flower *(Carthamus tinctorius),* beet *(Beta vulgaris),* sugarcane *(Saccharum officinarum),* cotton *(Gossypium spp), marijuana(Cannabis sativa),* jiangnan rolling cypress *(Selaginella moellendorffii),* lemon eucalyptus *(Corymbia citriodora),* water green tree *(Tetracentronsinense),* mesquite tree *(Prosopis alba),* taxus chinensis *(Taxus chinensis),* olive *(Olea europaea),* African acacia*(Abrusprecatorius),* switchgrass *(Panicum virgatum),* red-vein maiguo *(Rhamnellarubrinervis),* American hickory *(Carya illinoinensis),* tea *(Camellia sinensis),* tobacco *(Nicotiana tabacum),* elephant grass *(Pennisetum purpureum),* hard-straight ryegrass *(Lolium rigidum),* nandi *(Miscanthuslutarioriparius),* canweed *(Setariaviridis),* cockweed *(Pennisetum alopecuroides),* Sudan grass *(Sorghum sudanense),* Tang pine grass *(Thalictrum thalictroides),* curved leaf thrush *(Eragrostiscurvula),* Brachypodium *(Brachypodiumdistachyon),* alfalfa *(LotuscorniculatusL),* Arabidopsis *(Arabidopsis thaliana),*Physcomitrella*(Physeomitrium patens),* hornmoss *(Ceratodonpurpureus),* cabbage *(Brassica oleracea),* mustard *(Brassica juncea),* Chinesegreen cabbage *(Brassica rapa),* cauliflower *(Brassica oleracea),* lettuce *(Lactuca sativa),* spinach *(Spinacia oleracea), radish(Raphanus sativus),* Chinesecabbage *(Brassica rapa),* zucchini *(Cucurbita pepo),* hemsley *(Apium graveolens),* leek *(Allium tuberosum),* carrot *(Daucus carota),* eggplant*(Solanum melongena),* tomato *(Lycopersicon esculentum),* cucumber *(Cucumis sativus),* wax gourd *(Benincasahispida),* pumpkin *(Cucurbita moschata),* loofah *(Luffa cylindrica),* vegetable melon *(Cucumis melo),* lotus vegetables *(Potentilla anserina),*day lily *(Hemerocallis citrina),* stem lettuce *(Lactuca sativa),* asparagus *(Asparagus officinalis),* chili pepper *(Capsicum annuum),* garlic *(Allium sativum),* spring onion *(Allium fistulosum),* Jerusalem artichoke *(Helianthus tuberosus),* Coriander *(Coriandrum sativum),* watermelon *(Citrullus lanatus),* pear *(Pyrus spp),* peach *(Prunus persica),* apricot *(Armeniaca vulgaris),* lee *(Prunus salicina),* date *(Ziziphus jujuba),*green plum *(Vaticamangachapoi),* apple *(Malus pumila),* sand fruit *(Malus asiatica),* cherry *(Cerasus spp),* walnut *(Juglans regia),* strawberry *(Fragaria ananassa),* grape *(Vitis vinifera), pineapple* pear *(Ananas comosus),* coptis chinensis *(Coptis chinensis),* Chinese milk vetch *(Astragalus sinicus),* ginseng *(Panax ginseng),* Angelica sinensis *(Angelica sinensis),* honeysuckle *(Lonicera japonica),* Ai *(Artemisia argyi),* peppermint *(Mentha canadensis),* orchid *(Cymbidium ssp),* lily *(Lilium brownii),* tulip *(Tulipa gesneriana).*

### Example 2: A method of inducing haploids using OsCPRO1 and OsCPRO2

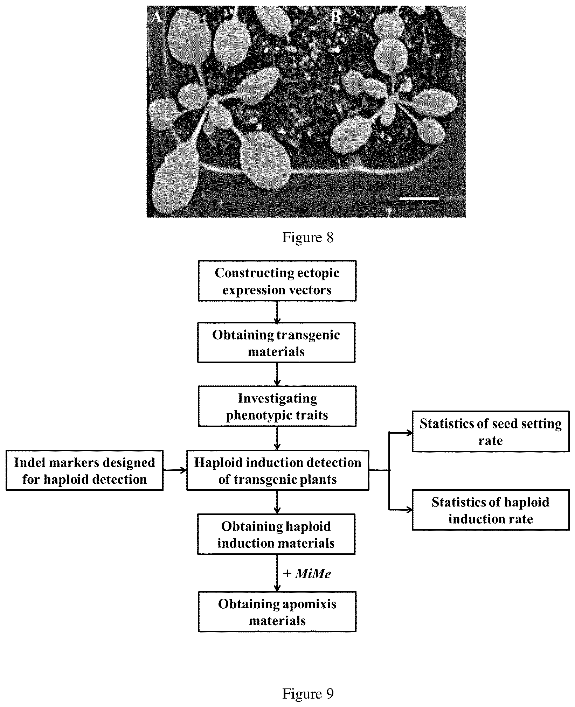

The method of inducing haploid by *OsCPRO1* and *OsCPRO2* is summarized as follows (Figure 9): ① selects the *AtDD45* promoter specifically expressed in egg cells, and the nucleotide sequence, as shown in SEQ ID NO: 7, drives the CDS sequence or genome sequence of *OsCPRO1* and *OsCPRO2* genes respectively to construct ectopic expression vector; ② uses Agrobacterium-mediated method to obtain transgenic-positive material; ③ investigates field phenotypic traits ofwild-type CY84 and T₀ transgenic-positive plants; ④ Indel markers designed for haploid detection; ⑤ statistics seed setting rate of T₀trans-positive material and germination treatment to obtain T₁generation of transgenic plants; ⑥ using the Indel markers against T₁generation of gene positive material; ⑦ statistics haploid induction rate of Titransgenic plants; ⑧ combines haploid induction material with the *MiMe* system to obtain apomixis material. The technical route profile is shown in Figure 9.

### Embodiment 1

### 1. Experimental materials

In this embodiment, the indica japonica hybrid rice Chunyou 84 (CY84) was selected as the genetic transformation material, CY84 is a hybrid breeding of the japonica sterile line Chunjiang 16A as the female parent and the indica restoring line C84 as the male parent, the hybrid rice has a longer growth period and is suitable for single season late rice cultivation. It has strong growth and large panicles with many grains. Due to its thick and robust stem, it has strong lodging resistance.

### 2. Construction of the ectopic expression vector

Select *AtDD45* oocyte specific expression promoter to drive the CDS sequences of *OsCPRO1* and *OsCPRO2* genes, respectively, to complete the ectopic expression process.

### 2.1 Get the fragments required for the ectopic expression vector constructs

(1) Arabidopsis leaf genomic DNA was used as a template to amplify the *AtDD45* promoter sequence.
   ①DNA extraction method: take about 2 cm of rice leaves into a 2 mL centrifuge tube with steel beads, 500 µL of CTAB extract was added; ② Transfer the centrifuge tube to the histiocyte shatter for shock grinding, 60 Hz/sec, 2 min; (3) Place the grinding samples in a 65°C water bath for 30 min, during this period, flip up and down for 10 min / time; ④ After taking out the sample, centrifuge at 12000 rpm for 30 seconds, then add 300 µL of chloroform to the fume hood and mix upside down. ⑤ Centrifuge again at 12000 rpm for 8 minutes. After centrifugation, take 350 µL of the supernatant and place it into a 1.5 mL sterilized centrifuge tube. Add 700 µL of precooled anhydrous ethanol, shake well, and transfer to a -20 °C freezer for 15 minutes. ⑥ After allowing the sample to stand still, centrifuge at 12000 rpm for 10 minutes. Discard the supernatant and air dry at room temperature. ⑦ Add 100 µL of ddH₂O for dissolution, gently shake, and store at 4 °C in the refrigerator for later use.
(2) RNA was extracted from CY84 and subsequently reverse transcribed into cDNA to provide a template for amplification of the CDS sequences of genes *OsCPRO1* and *OsCPRO2.*

Methods for RNA extraction: ① Put 3-4 cm young rice panicles into a sterilized mortar pre cooled with liquid nitrogen, grind clockwise until they turn into a white green powder, and quickly transfer them to an EP tube without RNase containing 1 mL of Trizol. Vortex and shake for 1 minute to fully mix; ② Incubate at room temperature for 5 minutes, add 200 µL chloroform to the EP tube, vortex for 15 seconds, and let it stand on ice for 2 minutes; (3) centrifuge at 12000 rpm at 4°C for 15 minutes; ④ Take 500 µL of supernatant, add 100 µL of chloroform, vortex for 15 seconds, and let it stand on ice for 2 minutes; ⑤ centrifuge at 12000 rpm at 4°C for 15 minutes; ⑥ Take 400 µL of the supernatant into a 1.5 mL EP tube without RNase, add 500 µL of isopropanol, invert and mix well; ⑦ Let it stand on ice for 10 minutes, centrifuge at 12000 rpm at 4°C for 10 minutes, and discard the supernatant; ⑧ Add 1 mL of 75% ethanol, wash the precipitate with vortex shaking, centrifuge at 12000 rpm at 4°C for 10 minutes,discard the supernatant and repeat the previous step; ⑩ Retain the RNA precipitate at the bottom of the tube, remove the residual liquid, simply air dry, add 100 µL of RNase free water, dissolve with light tap, and test the concentration and purity. Store at -80 °C for later use.

Methods for RNA reverse transcription into cDNA: preparation of cDNA by HiFiScript cDNA Synthesis Kit (CW2569M).① The reagent and RNA template provided in the kit were dissolved on ice for standby; ② mixed the reagent according to RNA reverse transcription system (Table 1), briefly centrifuged the liquid at the bottom of the tube; ③ Subsequently, the reaction procedure was carried out, with 15 minutes at 42°C followed by 5 minutes at 85°C; ④ Cool the cDNA on iceafter the reaction for later use.

**Table 1. System in which the RNA is reverse-transcribed into cDNA**

| Reagent | Addition |
|---|---|
| RNA template | 3 µg |
| Primer Mix | 2 µL |
| dNTP Mix | 4 µL |
| DDT | 2 µL |
| 5×RT Buffer | 4 µL |
| HiFiScript | 1 µL |
| RNase-Free Water | Fill it up to 20 µL |

(3) Design of the primers for amplification. The primers for the *AtDD45* promoter sequence and the CDS sequences of the *OsCPRO1* and *OsCPRO2* genes are shown in Table 2, with lower case letters indicating adaptor primers.

**Table 2. Primers for the ectopic expression vector constructs**

| Primer name | Sequence |
|---|---|
| AtDD45-F | tacgaattcgagctcggtacAAATGTTCCTCGCTGACGTAAGAAG |
| AtDD45-R | ACTTGTGTTAGAAGCCATTATTC |
| CPRO1-F | gaataatggcttctaacacaagtATGGCTACTTACTACTCGAGCCCTGGCAATG |
| CPRO1-R | catgcctgcaggtcgactctagagTCATCGAACCCACAGAGAAGCCATAG |
| CPRO2-F | gaataatggcttctaacacaagtATGGCTACTTACTATTCAAGCCCTGGTAGCG |
| CPRO2-R | catgcctgcaggtcgactctagagTCAGGCCACAAAATCATGCAGAAGAGGTG |

(4) The promoter sequences and the gene CDS sequences were amplified using the amplification primers. The amplification system is shown in Table 3, and the amplification procedure is shown in Table 4.

**Table 3. Amplification system of the fragments required for the vector construction**

| Reagent | Addition |
|---|---|
| KOD FX buffer | 25 µL |
| dNTP | 10 µL |
| KODFX | 1 µL |
| DNA template | 2 µL |
| Primer-F (10 µM) | 1.5 µL |
| Primer-R (10 µM) | 1.5 µL |
| ddH₂O | 9 µL |

**Table 4. Amplification procedure of the fragments required for the vector construction**

| Cycle index | Temperature setting and reaction time |
|---|---|
| 1 | 94°C 2 min |
| 35 | 98°C 10 sec, 60°C 30 sec, 68°C 1 min/kb |
| 1 | 68°C5 min |

(5) 1% agarose gel electrophoresis, cut the gel and save it at-20°C.

### 2.2 Skeleton vector enzyme digestion

The pCAMBIA1300-ACTIN skeleton vector was digested with KpnI-HF and Bam HI-HF endonuclease, removing the original ACTIN promoter sequence, forming a gap at the sticky end, and retaining all other sequences including the original terminator. The restriction system is shown in Table 5.

**Table 5. Enzyme digestion system of skeleton vector**

| Reagent | Addition |
|---|---|
| pCAMBIA1300-ACTIN | 2 µg |
| Cutsmart buffer | 5 µL |
| Kpn I-HF | 0.5 µL |
| Bam HI-HF | 0.5 µL |
| ddH₂O | Fill it up to 50 µL |

### 2.3 The recovered amplified fragments were ligated to the skeleton vector

The ligation system is shown in Table 6. The mixing system is placed at 50°C for 15 min. After ligation, the product is placed on ice.

**Table 6. Ligation system of the overexpression vectors**

| Reagent | Addition |
|---|---|
| Products digested from pCAMBIA1300-ACTIN *AtDD45* promoter of the amplified fragments Gene fragments amplified from the CDS sequences Gibson Assembly Master Mix (2×) ddH₂O | 2 µL |
| | 0.2 µg |
| | 0.4 µg |
| | 10 µL |
| | Fill it up to 20 µL |

### 2.4 Transformation of the ligation products

Method of transforming the ligation products: ① Remove the competent cells stored in the-80°C refrigerator (provided by the laboratory) and put them on ice for dissolution; (2) When the competent cell (50 µL ~100 µL) reaches the molten state, add the ligation products immediately; (3) Set still on the ice for 30 min; ④ 42°C water bath heat shock 90 sec; ⑤ 500 µL of liquid LB medium was added; ⑥ 37°C shaker culture for 1 h; ⑦ 4000 rpm, 3 min, slowly centrifuge and apply onto LB solid culture medium with Kan resistance; ⑧ Incubate overnight at 37 °C and perform colony PCR detection after the plaque grows. Select a single colony and culture it in Kan resistant liquid medium on a constant temperature shaker at 200 rpm and 37 °C for 14 hours. Extract plasmids and perform Sanger sequencing.

### 3. Transgenic plants obtaining

The ectopic expression vector was transferred into *Agrobacterium tumefaciens* strain EHA105 by electroshock and transferred into CY84 in hybrid rice using Agrobacterium-mediated method.

Specific method of transformation: ① Sterilize the embryos of hybrid rice CY84 seeds; ② Inoculate into the culture medium for inducing callus tissue; (3) After one week of cultivation, select embryogenic callus tissue that is vigorous in growth, light yellow in color, and relatively loose as the receptor for transformation; ④ Infect rice callus tissue with EHA105 strains containing pC1300-AtDD45P-OsCPRO1 and pC1300-AtDD45P-OsCPRO2 plasmids respectively; ⑤ Incubate in the dark at 25 °Cfor 3 days; @ Screening resistant callus tissues and transgenic plants using a selective culture medium containing 50mg/L hygromycin; ⑦ Select the transgenic plants for normal growth.

### 4. Plant the transgenic plants

The transgenic plants are planted in transgenic experimental fields in summer and in greenhouses in winter. The average temperature during the day in the greenhouse is 34 °C, the average temperature at night is 25 °C, there is a 12 hour/12 hour dark cycle, and the relative humidity is 75%.

### 5.Transgenic plants detection

### 5.1 Design of the primers for detection

Primers designed for the *AtDD45* promoter sequence and the gene CDS sequence (spanning the intron region) were also used as detection primers for transgenic positive plants. The specific primer information is shown in Table 7.

**Table 7. Primers for the detection of the transgenic plants**

| Primer name | Sequence | CDS amplification length | DNA amplification length |
|---|---|---|---|
| AtDD45-F2 | CTGATCTAGATGATGGTTATAGACTG | | 588 bp |
| AtDD45-R2 | ACTTGTGTTAGAAGCCATTATTC | | |
| CPRO1-F1 | CAATCTGAGGCCTCACAGGCAATCAG | 398 bp | 1154 bp |
| CPRO1-R1 | GTCTGAAGGGCCACTGCAGTATAAGGC | | |
| CPRO2-F1 | GATGAGGTCGTGAGTGTTTGGAAGAGC | 690 bp | 1121 bp |
| CPRO2-R1 | GACACGGGCATTTATGAACCAATTC | | |

5.2 The detection system is shown in Table 8 and the detection procedure is shown in Table 9.

**Table 8. Detection system of the transgenic plants**

| Reagent | Addition |
|---|---|
| 2×Rapid Taq Master Mix | 7.5 µL |
| DNA template | 1 µL |
| Primer-F (10 µM) | 0.5 µL |
| Primer-R (10 µM) | 0.5 µL |
| ddH₂O | 5.5 µL |

| Table 9. Detection procedures for the transgenic plants | |
|---|---|
| Cycle index Temperature setting and reaction time | |
| 1 | 95 °C 3 min |
| 35 | 95°C 15 sec, 60°C 15 sec, 72°C 15 sec |
| 1 | 72°C5 min |

### 6.Investigation of the field phenotype

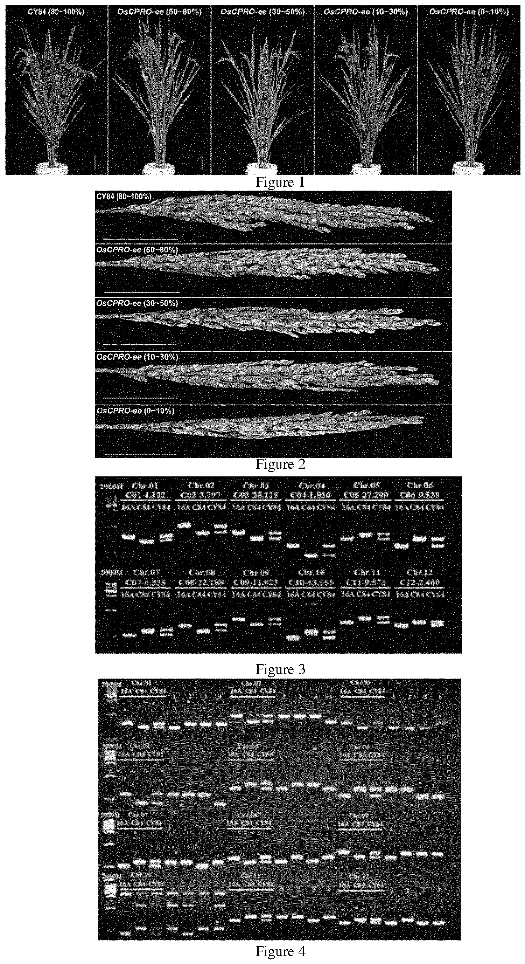

Field phenotype survey includes: plant type, ear type and seed set rate.
6.1 Mutant strain of plant type (Figure 1) and spike type (Figure 2) versus wild-type CY84 (n=3).
6.2 Statistics of seed set rate: 3 rice ears per plant were selected for statistics, and the calculation formula: seed set rate = (real grain number / total grain number) 100%.

### 7.Haploidsscreened using the Indel markers

According to the whole genome sequence of hybrid rice CY84 and its two parents (16A and C84), a pair of Indel markers were designed on each chromosome of rice, a total of 12 pairs of primer markers, primer information is shown in Table 10, genotyping is shown in Figure 3 for detection of haploid induction rate in T₁ generation transgenic materials. If all 12 pairs of Indel markers show the same single band genotype as the parent 16A or C84, rather than the same double band genotype as the heterozygous CY84, the probability of being haploid or double haploid can reach 99.98%, calculated as follows: 1- (1 / 2)¹²=99.98%. The Indel marker detection of T₁ haploid and double haploid materials is shown in Figure 4.

**Table 10. Indel markers used to detect haploids**

| Chromosome | Primer name | Sequence | Amplification length |
|---|---|---|---|
| Chr.01 | C01-4.122-F | GTGGTCAGGTGGTGATGGTGTT | 156 bp |
| | C01-4.122-R | AAAGAAACACGCAAATAAAAGC | |
| Chr.02 | C02-3.797-F | AACCTACCACTGCCATTGC | 202 bp |
| | C02-3.797-R | GGCATTATCCATACCAGCAG | |
| Chr.03 | C03-25.115-F | ACATGGCCTTGTAGTAGACGAGAG | 204 bp |
| | C03-25.115-R | ACGCTGTGGCTATGCCTTTGG | |
| Chr.04 | C04-1.866-F | ACCATGCCTCATGACATGTGG | 128 bp |
| | C04-1.866-R | TGGTTTTGTGTAGCTCTGTCGG | |
| Chr.05 | C05-27.299-F | ACAGCGATAATAACACGCACAA | 163 bp |
| | C05-27.299-R | TCAAGTGCTATACTTGACACGG | |
| Chr.06 | C06-9.538-F | CCATAAGATGCAGGCCGTTGT | 129 bp |
| | C06-9.538-R | CAGCTTTGGTCAGATGGTCAC | |
| Chr.07 | C07-6.338-F | GATTTATAGTTTGAGTGTTTGC | 125 bp |
| | C07-6.338-R | CTTGGTTAGTTTCTACCCTGCT | |
| Chr.08 | C08-22.188-F | CATGCAGATAGCTCGCTTGT | 143 bp |
| | C08-22.188-R | CACCTCTCAGGACAACTGTA | |
| Chr.09 | C09-11.923-F | TTCATCCCAGCCTACCTCCT | 174 bp |
| | C09-11.923-R | GCTTAATCCCGTAGTCTTCAA | |
| Chr.10 | C10-13.555-F | GCACATGGTGAGACGTCCTC | 103 bp |
| | C10-13.555-R | AAGTCCTGTAGTAGGTCACACCG | |
| Chr.11 | C11-9.573-F | GGCATCATTAAGGCTTGT | 160 bp |
| | C11-9.573-R | CTGGCGATCTCTGTGAGG | |
| Chr.12 | C12-2.460-F | GAGCAGATCACCCCTAAATTATG | 150 bp |
| | C12-2.460-R | GATTCATTCATCTTTCGAAGAG | |

### 8. Flow-cytometric ploidy detection

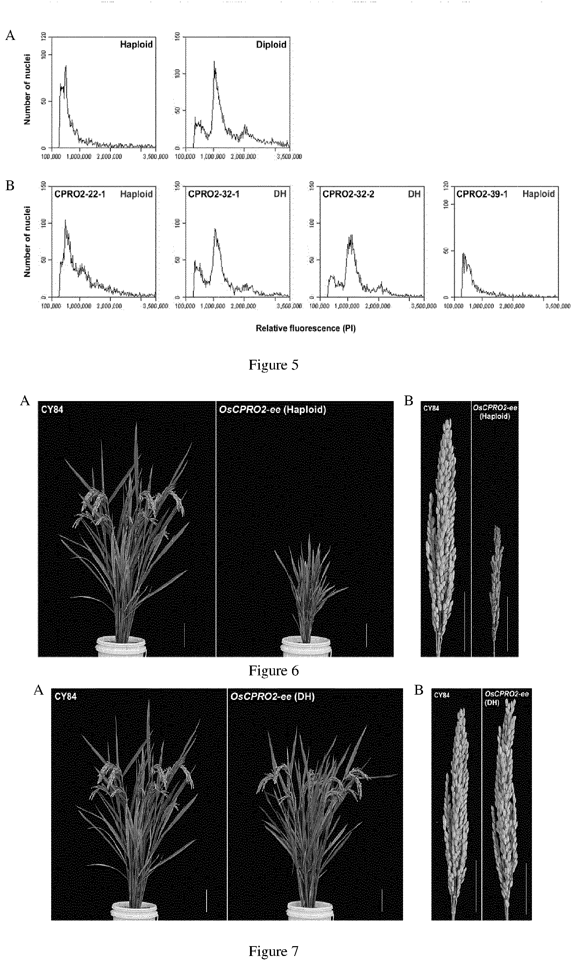

The haploid or diploid plants screened by Indel markers were further confirmed for cell ploidy using flow cytometry (Figure 5). The comparison of traits between haploid materials and wild-type CY84 is shown in Figure 6, while the comparison of traits between double haploid materials and wild-type CY84 is shown in Figure 7.

The method of flow cytometry ploidy detection: ① Cut about 3 cm of fresh young leaves and place them in sterilized glass petri dishes. Place the petri dishes in a tray filled with ice, and perform the following on ice; ② Add 1 mL of plant lysis buffer (LB01) to the petri dish, cut the leaves with a blade, and immerse the tissue cells of the leaves into the lysis buffer; (3) Extract the lysis solution from the petri dish, filter it with 50 µm nylon mesh into a 1.5 mL sterile centrifuge tube, and store it on ice. Centrifuge at 1200 rpm at 4 °Cfor5 minutes; ⑤ Discard the supernatant and add pre cooled 450 µL of LB01, 25 µL of PI (1 mg/mL), and 25 µL of RNaseA (1 mg/mL); ⑥ Staining on ice in the dark for 10 minutes; ⑦ Detecting with BD Accuri C6 machine.

### 9.Whole-genome sequencing analysis

Select the parents 16A and C84 of hybrid rice CY84, and CY84, as well as the leaves of haploid plants, and extract DNA for whole genome sequencing. The whole genome sequencing results show that there are many different homozygous genotypes between 16A and C84; The genotype of CY84 at these loci is a heterozygous genotype that includes both the 16A genotype and the C84 genotype; while the haploid plant at these loci with homozygous genotypes of 16A or C84.

### Embodiment 2

**1. Experimental materials:** see embodiment 1
**2. Ectopic expression vector construction**
   The *AtDD45* egg specific expression promoter was selected to drive the genomic sequence of *OsCPRO1* and *OsCPRO2* genes respectively to complete the ectopic expression process.
   2.1 Get the fragments required for the ectopic expression vector constructs
      (1) Genomic Arabidopsis leaf DNA was used as a template to amplify the *AtDD45* promoter sequence. For DNA extraction, refer to the described in embodiment 1.
      (2) Genomic DNA extracted from rice leaves was used as a template to amplify the genomic sequence of the *OsCPRO1* and *OsCPRO2* genes.
      (3) The amplification primers were designed and used to amplify the *AtDD45* promoter sequence and the *OsCPRO1* and *OsCPRO2* genomic sequences.
      (4) 1% agarose gel electrophoresis, cut the gel and save it at-20°C.
   2.2 Skeleton vector enzyme digestion: see embodiment 1.
   2.3 Ligate the recovered amplified fragment to the skeleton vector: see embodiment 1.
   2.4 Transformation and ligation products: see embodiment 1.
**3. Get transgenic plants:** see embodiment 1.
**4. Plant transgenic plants:** see embodiment 1.
**5.Transgenic plants detection**
   Primers were designed on the *AtDD45* promoter sequence for detection of transgenic-positive plants. See primer information in Table 7 in embodiment 1, Table 8 in embodiment 1 for detection system, and Table 9 in embodiment 1 for amplification procedures.
**6. Investigate field phenotypes:** see embodiment 1.
**7. Haploid screening using Indel markers:** see embodiment 1.
**8. Flow cytometric ploidy detection:** see embodiment 1.
**9. Whole-genome sequencing analysis:** see embodiment 1.

### Embodiment 3

### 1.Experimental materials: wild ArabidopsisColumbia (Col-0) ecotype was used as the experimental materials.

### 2. Ectopic expression vector construction

The eggspecific expression promoter of *AtDD45* was selected to drive the CDS sequences of *AtCPRO1* and *AtCPRO2* genes respectively to complete the ectopic expression process.
2.1 Get the fragments required for the ectopic expression vector constructs
   (1) Arabidopsis leafGenomic DNA was used as a template to amplify the *AtDD45* promoter sequence. For DNA extraction, described in embodiment 1.
   (2) RNA was extracted from young flowers of the Arabidopsis Columbia ecotype and subsequently reverse transcribed into cDNA to provide a template for the amplification of the CDS sequences of the genes *AtCPRO1* and *AtCPRO2.* Methods for RNA extraction and RNA reverse transcription into cDNA see embodiment 1.
   (3) The amplification primers were designed and amplified using the *AtDD45* promoter sequence and the CDS sequences of the *AtCPRO1* and *AtCPRO2* genes. The amplification system is shown in Table 3 in embodiment 1 and the amplification procedure is shown in Table 4 in embodiment 1.
   (4) 1% agarose gel electrophoresis, cut the gel and save it at-20°C.
2.2 Skeleton vector enzyme digestion: see embodiment 1.
2.3 Ligate the recovered amplified fragment to the skeleton vector: see embodiment 1.
2.4 Transformation and ligation products: See embodiment 1.

### 3. Transgenic plants obtaining

The ectopic expression vector was transferred into *Agrobacterium tumefaciens* strain GV 310 by electroshock and transferred into Col-0 ecotype wild *A*. *thaliana* using Agrobacterium-mediated dipping method.

Specific method of transformation: ① Immerse the inflorescence of T₀ generation Arabidopsis in a suspension of *Agrobacterium tumefaciens* strain GV310 containing the target transformation plasmid at a certain concentration; (2) Cultivate under certain conditions; (3) Collect seeds of T₀ generation Arabidopsis after maturity; ④ Place these seeds on a culture medium containing specific antibiotics for growth screening and obtain positive plants.

### 4. Plant the transgenic plants

The transgenic material was grown in a light incubator. Light treatment for 15 hours, with an average temperature of 24 °C; dark treatment for 9 hours, with an average temperature of 22 °C. The average humidity of the soil is 75%, and the nutrient soil: vermiculite=1:1. Water the nutrient solution every 3-4 days and immerse it from the bottom of the plate.

### 5.Transgenic plants detection

Design primers on the CDS sequence of genes, spanning intron regions, and use this primer as a detection primer for transgenic positive plants. The detection system can be found in Table 8 of embodiment 1, and the amplification program can be found in Table 9 of embodiment 1.

### 6.Investigation of the phenotypic traits

Phenotypic trait surveys included plant type and seed set rate.
6.1 Investigation of the plant type of the T₂ generation (Figure 8), selecting mutant plants and wild-type Arabidopsis for comparative observation (n=3).
6.2 Statistics of Seed Setting Rate: Collect mature but not yet cracked wild type and mutant Arabidopsis longhomed fruits, put them into a decolorization solution (ethanol: acetic acid=3:1) for transparent decolorization, and when the seeds in the pods can be clearly seen, take them out and place them under a microscope to calculate the seed setting rate.

### 7. Flow-cytometric ploidy detection

Confirm the cell ploidy of plants using flow cytometry technology, and refer to embodiment 1 for the method of flow cytometry ploidy detection.

### Embodiment 4

**1. Experimental materials:** see embodiment 1.
**2. Construct an ectopic expression vector:** see embodiment 1.
**3.Multigene knockout vectors construction**
MiMe material was constructed by using the CRISPR-Cas 9 multigene knockout system to simultaneously knockout *OsOSD1, OsPAIR1* and *OsREC8* rice endogenous genes, with the main steps as follows (see CN201510485573.2 for specific operational details):
3.1 Design of the target sequences and the primers
(1) Design of target sequence: search for NNNNNNNNNNNNNNNNNNN (NGG) specific sequence at the target position of the gene, with GC content between 35% and 75%. The following four sites were selected as sites for knockout *OsOSD1, OsPAIR1* and *OsREC8* by CRISPR-Cas 9 gene editing system (underlined PAM sequence):
   Knockdown site of the *OsOSD1* gene: CTGCCGCCGACGAGCAACA AGG
   Knockdown site of the *OsPAIR1* gene: AAGCAACCCAGTGCACCGC TGG
   Knockdown site of the *OsREC8* gene: CGGAGAGCCTTAGTGCCAT GGG
(2)Primer design: add four bases of GGCA before the forward target sequence and four bases of AAAC before the reverse target sequence, respectively named g⁺⁺and g⁻⁻, performed the primer synthesis.

3.2 Construction of the intermediate vector
(1) The Aar I enzyme was used to digest the intermediate vector SK-gRNA to form the sticky ends, and the digestion system is shown in Table 11.

**Table 11.Enzyme digestion system of the intermediate vector SK-gRNA**

| Reagent | Addition |
|---|---|
| SK-gRNA | 2 µL |
| 10 × buffer Aar I | 5 µL |
| 50 × oligonucleotide | 1 µL |
| *Aar I* | 1 µL |
| ddH₂O | 41 µL |

(2) Mix 20 µL of 100 µM g++and g-- primers in equal proportions, and incubate at 100°C for 5 minutes to form sticky ends after denaturation annealing.
(3) Ligating via T4 DNA ligase and ligate at room temperature for 1 hour. The ligation system is shown in Table 12.

**Table 12.The ligation system of the DNA ligase**

| Reagent | Addition |
|---|---|
| SK-gRNA /Aar I | 20-50 ng |
| The primer was annealed to the product | 7 µL |
| 10 × T₄ligase buffer | 1 µL |
| T₄DNA ligase | 1 µL |
| ddH₂O | Fill it up to 10 µ L |

(4) Transformation ligation products: see embodiment 1.
3.3 Construct the final vector
Utilizing the properties of BamHI and BglII being homologous enzymes, the polymerization of three SK- gRNA intermediate vectors was completed using T4 DNA ligase. As the final vector, KpnI and BamHI were used for digestion, while KpnI and BglII were used for digestion of the fragments provided. The ligation between SK- gRNAs was completed using two pairs of homologous enzymes NheI/XbaI and SalI/XhoI. The system with multiple intermediate vectors ligated is shown in Table 13.

**Table 13 Ligation systems of multiple intermediate vectors**

| Reagent | Addition |
|---|---|
| pC1300-Ubi-Cas9/Kpn I+Bam HI | 50 ng |
| gRNA1/Kpn I+Sal I | 8 ng |
| gRNA2/XhoI+NheI | 8 ng |
| gRNA3/XbaI+BglII | 8 ng |
| 10 × T₄ligase buffer | 1 µL |
| T₄DNA ligase | 0.5 µL |
| ddH₂O | Fill it up to 10 µL |

**4. Binary fusion expression vector construction**

The ectopic expression vector and the multigene knockout vector were combined to construct a binary fusion expression vector. Among them, the ectopic expression vector provided the desired fragment, and the multigene knockout vector was used as the backbone vector for enzyme digestion.

### 4.1 Get the fragments required for the construction of the binary fusion expression vector

The whole fragment of the ectopic expression process was amplified from the ectopic expression vector, including three parts: *AtDD45* promoter fragment, *OsCPRO1* and *OsCPRO2* gene CDS sequence and terminator sequence. The amplification primers are shown in Table 14 and lower letters indicate the adaptor primers.

**Table 14. Amification primers for the construction of binary fusion expression vector**

| Primer name | Sequence |
|---|---|
| CPRO-F | gattgtcgtttcccgccttcagtttAAATGTTCCTCGCTGACGTAAGAAG |
| CPRO-R | CGCCAATATATCCTGTCAAACACTGATAGTTT |

### 4.2 Digestion of multiple gene knockout vectors

Using PmeI endonuclease, the pC1300-Cas9-gRNAOSD1-gRNAPAIR1-gRNAREC8 multi gene knockout vector was digested. The digestion system is shown in Table 15.

**Table 15. Multigene knockout vector enzyme digestion system**

| Reagent | Addition |
|---|---|
| pC 1300-Cas9-gRNA^{OSD1}-gRNA^{PAIR1}-gRNA^{REC8} | 20n g |
| Cutsmart buffer | 5 µ L |
| *Pme I* | 1µ L |
| ddH₂O | Fill it up to 50 µ L |

### 4.3 The amplified fragment was attached to the multigene knockout vector

The ligation system is shown in Table 16. The mixing system is placed at 50°C for 15 min, and the product is placed on ice after the ligation.

**Table 16. Ligation system of the binary fusion expression vectors**

| Reagent | Addition |
|---|---|
| pC 1300-Cas9-gRNA^{OSD1}-gRNA^{PAIR1}-gRNA^{REC8}/*Pme I* | 2 µL |
| AtDD45 Promoter-OsCPRO1 (CDs) / OsCPRO2 (CDs) - terminator amplified fragment | 4µL |
| Gibson Assembly Master Mix (2×) | 10 µL |
| ddH₂O | Fill it up to 20 µL |

### 4.4 Transformation of the ligation products: See embodiment 1.

### 5. Get transgenic plants: see embodiment1.

### 6. Plant transgenic plants: see embodiment1.

### 7.Transgenic plants detection

7.1 Positive detection of transgenic plants: see embodiment 1.
7.2 Detection of the knockdown of the target sites
(1) Primers were designed for target site detection to determine the knockdown of *OsOSD1, OsPAIR1* and *OsREC8* genes. The primer information is shown in Table17. Lower case letters indicate the joint primers.

**Table 17.Primers for OsOSD1, OsPAIR1 and OsREC8 knockdown**

| Primer name | Sequence |
|---|---|
| OSD1-F | ggagtgagtacggtgtgcTATCAGGAGGACGACGTCGCCG |
| OSD1-R | gagttggatgctgagtggCTCCTCCTCTTGGGTGTAGC |
| PAIR1-F | ggagtgagtacggtgtgcCTTCTTGCGCGCGAGAAGAGTCTC |
| PAIR1-R | gagttggatgctgagtggGAGATGTAGTGCGTGGGTCTTG |
| REC8-F | ggagtgagtacggtgtgcTTGGGTTAGTGAGGAGAT |
| REC8-R | gagttggatgctgagtggTGCGATCGGAACTATGGAGAC |

(2) sequencing analysis. The PCR amplification products were sent to the China Rice Research Institute for second-generation sequencing, using Hi-TOM (http://www.hi-tom. The net / hi-tom /) platform performed data analysis to obtain mutational information for the three genes.

### 8. Investigate field phenotypes: see embodiment1.

### 9. Flow-cytometric ploidy detection

### 9.1 Material preparation

The materials with successful binary fusion expression were selected, that is, when the *AtDD45* promoter drives the successful expression of the CDS sequence of the *OsCPRO1* / *OsCPRO2* gene, *OsOSD1, OsPAIR1* and *OsREC8* were successfully knocked out (MiMe). The seeds of this material are germinated and planted (first filial generation).

### 9.2 Flow cytometry

We screened the cell ploidy by flow cytometry to select the first filial generation plants with consistent cell ploidy and the parent plant as apomixis material.

### 10. Analysis of whole-genome sequencing: see embodiment 1.

### Embodiment 5

**1. Experimental materials:** see embodiment 1.
**2. Construction of an ectopic expression vector:** see embodiment 2.
**3. Construction of multigene knockout vectors:** see embodiment 4.
**4.Construct a binary fusion expression vector**
   The ectopic expression vector and the multigene knockout vector were combined to construct a binary fusion expression vector. Among them, the ectopic expression vector provided the desired fragment, and the multigene knockout vector was used as the backbone vector for enzyme digestion.
   4.1 Get the fragments required for the construction of the binary fusion expression vector
      The genomic sequence of the *AtDD45* egg-specific expression promoter driving the *OsCPRO1* and *OsCPRO2* genes, completing the whole fragment of the ectopic expression process, was amplified from the ectopic expression vector, containing three parts: the *AtDD45* promoter fragment, the genomic sequence of the *OsCPRO1* and *OsCPRO2* genes, and the terminator sequence. The amplification primers are shown in Table 14 in embodiment 3.
   4.2 To digest multiple gene knockout vector: see embodiment 4.
   4.3 Attach the amplified fragments to the multigene knockout vector: see embodiment 4.
   4.4 Transformation of the ligation products: see embodiment 1.
**5. Get transgenic plants:** see embodiment1.
**6. Plant transgenic plants:** see embodiment 1.
**7.Transgenic plants detection**
   7.1 Positive detection of transgenic plants: see embodiment 2.
   7.2 Detection of target site knockout: see embodiment 4.
**8. Investigate field phenotypes:** see embodiment 1.
**9. Flow cytometric ploidy detection:** see embodiment 4.
**10. Analysis of whole-genome sequencing:** see embodiment 1.

### Example 3

### 1. Phenotypic traits of T₀generation OsCPRO series haploid-inducing material

For embodiment 1 and 2 in Example 2, investigation of field phenotypes of T₀transgenic positive plants revealed that compared with the wild-type CY84, the transgene positive material had no significant difference in vegetative growth, and the seed set rate was reduced to different degrees, including 80-100%, 50-80%, 50-30-50%, 10-30%, and 0-10%. The plant type was summarized in Figure 1 and the spike type was shown in Figure 2.

### 2. Genotyping of the 12 pairs of Indel markers

For embodiment 1 and 2 in Example 2,12 pairs of Indel markers used to screen haploids and double haploids showed obvious polymorphism and could be used for genotype identification of transgenic materials, as shown in Figure 3.

### 3. Genotyping and ploidy detection for haploid and double haploid material

For screening the haploid material or double haploid material in Example 2, the test results of the CDS sequence of *OsCPRO2* in Example 1:12 Indel marker sites showed single band genotype (Figure 4), which is haploid material or double haploid material. Two ploidy (CPRO 2-22-1 and CPRO 2-39-1) showed haplotype (n) genome and 2 (CPRO 2-32-1 and CPRO 2-32-2) showed diplotype (2n) genome as double haploid material (Figure 5).

### 4. Trait examination of haploid and double-haploid materials

For the haploid and double haploid materials selected from embodiment 1 and 2 in Example 2, taking the detection results of *AtDD45* oocyte specific expression promoter driving *OsCPRO2* to achieve ectopic expression in embodiment 1 as an example, compared with the wild-type CY84, haploid materials were significantly affected in terms of nutritional and reproductive growth: plant height decreased, spike length shortened, glume reduced, and fertility completely lost (Figure 6). Compared with the wild-type CY84, there was no significant difference in nutritional and reproductive growth between the double haploid material (Figure 7). Compared with the Col-0 ecotype wild *Arabidopsis thaliana,* the haploid material obtained from embodiment 3 in Example 2 was significantly affected in both nutritional and reproductive growth: the plant size decreased and the leaves narrowed (Figure 8).

### 5. Detection of the CPRO series of haploid induced materials and apomixis materials

For the T1 generation transgenic positive materials of embodiment 1, 2, and 3 in Example 2, it was found that the materials with haploid induction ability had a variation range of 18.98 ± 2.14%~50.88 ± 0.74% in seed setting rate and 0.44%~7.14% in induction rate. The specific statistical information is shown in Table 18. Testing was conducted on the T1 generation transgenic positive materials of embodiment 4 and 5 in Example 2, and it was found that the fusion free reproductive materials produced by ectopic expression of *OsCPRO1* and *OsCPRO2* genes in oocytes combined with the MiMe system ranged in proportion to cloned seeds from 0.95% to 7.92%. The specific statistical information is shown in Table 19.

**Table 18.CPRO detection of haploid-induced materials in the series**

| Experimental example | Genotype | Plant number | Setting percentage | Haploid or double haploid / total plant number (induction rate) |
|---|---|---|---|---|
| Rice wild type | WT | CY84 | 82.12±4.02% | 0 |
| Experiment case 1 | The CDS sequences of the ectopically expressed *OsCPRO1* gene | 4 | 22.48±1.50%** | 8/112 (7.14%) |
| | | 17 | 24.91±1.93%** | 5/240 (2.08%) |
| | | 20 | 27.35±3.02%** | 4/69 (5.80%) |
| | *The CDS sequences of the ectopically expressed OsCPRO2 gene* | 22 | 28.83±0.62%** | 1/76 (1.32%) |
| | | 32 | 19.94±1.94%** | 4/153 (2.61%) |
| | | 39 | 25.13±4.74%** | 1/226 (0.44%) |
| Experiment case 2 | Genomic sequence of the ectopically expressed OsCPRO1 gene | 19 | 25.73±1.97%** | 2/115 (1.74%) |
| | | 35 | 23.56±2.32%** | 5/102 (4.90%) |
| | | 36 | 28.87±0.92%** | 7/111 (6.31%) |
| | Genomic sequence of the ectopically expressed OsCPRO2 gene | 2 | 24.82±1.98%** | 2/95 (2.11%) |
| | | 11 | 18.98±2.14%** | 3/162 (1.85%) |
| | | 23 | 30.35±1.04%** | 2/326 (0.61%) |
| Wild type of Arabidopsis | WT | Col-0 | 84.25±3.23% | 0 |
| thaliana | | | | |
| Experimental case 3 | The CDS sequences of the ectopically expressed *At CPRO1* gene | 1 | 41.15±1.48%** | 1/100 (1.00%) |
| | | 9 | 32.08±1.76%** | 1/102 (0.98%) |
| | | 21 | 25.40±2.39%** | 2/96 (2.08%) |
| | The CDS sequences of the ectopically expressed *At CPRO2* gene | 3 | 33.34±1.57%** | 1/95 (1.05%) |
| | | 7 | 27.69±3.85 %** | 1/84 (1.19%) |
| | | 14 | 50.88±0.74%** | 1/116 (0.86%) |

| | | | | |
|---|---|---|---|---|
| Using wild-type CY84 as a control, the data were t-test, error lines indicate the standard deviation (n=3), * * P <0.01. | | | | |

**Table 19. Detection of CPRO series**

| Experimental example | Genotype | Plant number | Setting percentage | Number of cloned seeds / total grains (induction rate) |
|---|---|---|---|---|
| Control materials | WT | CY84 | 82.12±4.02% | 0 |
| Experimental case 4 | The CDS sequence of the ectopically expressed *OsCPRO1* genes is bound to *MiMe* | 5 | 29.20±1.67 %** | 3/96 (3.13%) |
| | | 11 | 19.84±2.43%** | 8/101 (7.92%) |
| | | 23 | 25.81±1.87%** | 3/86 (3.49%) |
| | The CDS sequence of the ectopically expressed *OsCPRO2* genes is bound to *MiMe* | 2 | 18.92±1.09%** | 3/80 (3.75%) |
| | | 21 | 21.45±0.56%** | 2/144 (1.39%) |
| | | 22 | 26.55±1.73%** | 2/211 (0.95%) |
| Experimental case 5 | The genomic sequence of ectopically expressed *OsCPRO1* genes binds to *MiMe* | 17 | 22.75±1.18%** | 4/105 (3.81%) |
| | | 32 | 28.90±0.44%** | 5/111 (4.50%) |
| | | 35 | 27.33±1.54%** | 2/104 (1.92%) |
| | The genomic sequence of ectopically expressed *OsCPRO2* genes binds to *MiMe* | 24 | 21.78±1.20%** | 1/65 (1.54%) |
| | | 31 | 19.65±2.28%** | 2/88 (2.27%) |
| | | 34 | 25.98±3.01%** | 1/94 (1.06%) |

| | | | | |
|---|---|---|---|---|
| Using wild-type CY84 as a control, the data were t-test, error lines indicate the standard deviation (n=3), * * P <0.01. | | | | |

## Claims

1. A protein with haploid induction ability, among which are the POX superfamily domain and the Homeobox domain, specifically, POX, the superfamily domain containing BELL domain and SKY domain: SKYLKAAQELLDEVVSV; preferably it is a OsCPRO1 or OsCPRO2 protein, amino acid sequences are shown as in SEQ ID NO: 3 and SEQ ID NO: 6, respectively, or their orthologous genes derived from the following species, optimally greater than 90%, 95%, more than 98% or 99%: rice *(Oryza sativa),* wart-grain rice *(Oryza meyeriana varY,* maize*(Zea mays),* wheat *(Triticum aestivum),* durum wheat *(Triticum turgidum subsp),* wild emmer wheat*(Triticum dicoccoides),* urartu wheat *(Triticum urartu),* barley *(Hordeum vulgare),* section wheat *(Aegilops tauschii),* rye *(Secale cereale),* oats *(Avena sativa),* buckwheat *(Fagopyrum esculentum),* highland barley *(Hordeum vulgare),* semen coicis *(Coixlacryma-jobi)*, foxtail millet*(Setariaitalica),* fonio millet *(Digitariaexilis),* sorghum *(Sorghum bicolor),* proso *millet(Panicum miliaceum),* wild rice *(Zizania latifolia),* biotic wild rice *(Zizania palustris), coracana(Eleusine coracana),* sweet potato *(Dioscorea esculenta),* cassava *(Manihot esculenta),* potato *(Solanum tuberosum),* yam*(Dioscoreapolystachya),* groundnut *(Arachis hypogaea),* cranes peanut *(Arachis duranensis),* soybean *(Glycine max),* wild soybean *(Glycine soja),* adzuki bean *(Vigna angularis),* red bean *(Vigna umbellata),* lentil bean *(Lens culinaris),* mung bean *(Vigna radiata),* cowpea *(Vigna unguiculata),* kidney bean *(Phaseolus vulgaris),* broad bean *(Vicia faba),* pea *(Pisum sativum),* jack bean *(Canavalia gladiata),*pigeonpea *(Cajanus cajan),* white lupine *(Lupinus albus),* lather bean*(Mucuna pruriens),* narrow-leafed lupine *(Lupinus angustifolius),* hairy vine bean *(Calopogoniummucunoides),* chickpea *(Cicer arietinum),* sesame *(Sesamum indicum),* flax *(Linumusitatissimum),* rapeseed *(Brassica napus),* sunflower *(Helianthus annuus),* castor bean *(Ricinus communis),* red flower *(Carthamus tinctorius),* beet *(Beta vulgaris),* sugarcane *(Saccharum officinarum),* cotton *(Gossypium spp), marijuana(Cannabis sativa),* jiangnan rolling cypress *(Selaginella moellendorffii),* lemon eucalyptus *(Corymbia citriodora),* water green tree *(Tetracentronsinense),* mesquite tree *(Prosopis alba),* taxus chinensis *(Taxus chinensis),* olive *(Olea europaea),* African acacia*(Abrusprecatorius),* switchgrass *(Panicum virgatum),* red-vein maiguo *(Rhamnellarubrinervis),* American hickory *(Carya illinoinensis),* tea *(Camellia sinensis),* tobacco *(Nicotiana tabacum),* elephant grass *(Pennisetum purpureum),* hard-straight ryegrass *(Lolium rigidum),* nandi *(Miscanthuslutarioriparius),* canweed *(Setariaviridis),* cockweed *(Pennisetum alopecuroides),* Sudan grass *(Sorghum sudanense),* Tang pine grass *(Thalictrum thalictroides),* curved leaf thrush *(Eragrostiscurvula),* Brachypodium *(Brachypodiumdistachyon),* alfalfa *(LotuscorniculatusL),* Arabidopsis *(Arabidopsis thaliana),*Physcomitrella*(Physcomitrium patens),* hornmoss *(Ceratodonpurpureus),* cabbage *(Brassica oleracea),* mustard *(Brassica juncea),* Chinesegreen cabbage *(Brassica rapa),* cauliflower *(Brassica oleracea),* lettuce *(Lactuca sativa),* spinach *(Spinacia oleracea), radish(Raphanus sativus),* Chinesecabbage *(Brassica rapa),* zucchini *(Cucurbita pepo),* hemsley *(Apium graveolens),* leek *(Allium tuberosum),* carrot *(Daucus carota),* eggplant*(Solanum melongena),* tomato *(Lycopersicon esculentum),* cucumber *(Cucumis sativus),* wax gourd *(Benincasahispida),* pumpkin *(Cucurbita moschata),* loofah *(Luffa cylindrica),* vegetable melon *(Cucumis melo),* lotus vegetables *(Potentilla anserina),*day lily *(Hemerocallis citrina),* stem lettuce *(Lactuca sativa),* asparagus *(Asparagus officinalis),* chili pepper *(Capsicum annuum),* garlic *(Allium sativum),* spring onion *(Allium fistulosum),* Jerusalem artichoke *(Helianthus tuberosus),* Coriander *(Coriandrum sativum),* watermelon *(Citrullus lanatus),* pear *(Pyrus spp),* peach *(Prunus persica),* apricot *(Armeniaca vulgaris),* lee *(Prunus salicina),* date *(Ziziphus jujuba),*green plum *(Vaticamangachapoi),* apple *(Malus pumila),* sand fruit *(Malus asiatica),* cherry *(Cerasus spp),* walnut *(Juglans regia),* strawberry *(Fragaria ananassa),* grape *(Vitis vinifera), pineapple* pear *(Ananas comosus),* coptis chinensis *(Coptis chinensis),* Chinese milk vetch *(Astragalus sinicus),* ginseng *(Panax ginseng),* Angelica sinensis *(Angelica sinensis),* honeysuckle *(Lonicera japonica),* Ai *(Artemisia argyi),* peppermint *(Mentha canadensis),* orchid *(Cymbidium ssp),* lily *(Lilium brownii),* tulip *(Tulipa gesneriana).*

2. The gene encoding a protein as described in claim 1, which is either*OsCPR01* or *OsCPRO2,* where the gene symbol of *OsCPRO1* is *LOC_Os03g47740,* the gene symbol of *OsCPRO2* is *LOC_Os12g43950*; Or BEL1-like homeodomain protein 6 derived from maize, Sequence ID: PWZ21223.1; Or BELl-like homeodomain protein 7 derived from wheat, Sequence ID: XP_044365192.1; Or BEL1-like homeodomain protein 1 derived from soybean, Sequence ID: XP_003543416.1; Or BEL1-like homeodomain protein 1 from groundpeanut, Sequence ID: XP_016170518.1; Or at least 90%, 95% of the above genes, more preferably more than 98%, or 99% of the homologous genes derived from the same species, and still has the haploid induction ability.

3. The gene with haploid induction ability as described in claim 2 is **characterized by** the nucleotide sequence of *OsCPRO1* as shown in SEQ ID NO: 1, or more than 90%, 95%, 98% and 99% of homologous genes derived from rice and still have haploid induction ability; the nucleotide sequence of *OsCPRO2* as shown in SEQ ID NO: 4, preferably more than 98% and 99% of homologous genes derived from rice, and still has haploid induction ability.

4. The gene having the haploid induction ability as described in claim 2, it is **characterized by** the fact that, the CDS nucleotide sequence of *OsCPRO1* is as shown in SEQ ID NO: 2, or is derived from rice that still has a haploid-inducing nucleotide sequence with greater than 98% or 99% identity, and still has the haploid induction ability; the CDS nucleotide sequence of *OsCPRO2* is as shown in SEQID NO: 5, or derived from rice still has haploid induction ability with more than 98% and 99% identity.

5. An expression cassette, a recombinant vector, a recombinant cell, or a host cell containing a gene according to claim 2 to 4.

6. The protein according to claim 1, or the application of the gene in the induced plant haploids as per any one of claim 2to 4, preplant from rice *(Oryza sativa),* wart-grain rice *(Oryza meyeriana var),* maize(*Zea mays),* wheat *(Triticum aestivum),* durum wheat *(Triticum turgidum subsp),* wild emmer *wheat(Triticum dicoccoides),* urartu wheat *(Triticum urartu),* barley *(Hordeum vulgare),* section wheat (*Aegilops tauschii),* rye *(Secale cereale),* oats (*Avena sativa),* buckwheat *(Fagopyrum esculentum),* highland barley *(Hordeum vulgare),* semen coicis (*Coixlacryma-jobi*), foxtail millet*(Setariaitalica),* fonio millet *(Digitariaexilis),* sorghum *(Sorghum bicolor),* proso *millet(Panicum miliaceum),* wild rice *(Zizania latifolia),* biotic wild rice *(Zizania palustris), coracana(Eleusine coracana),* sweet potato *(Dioscorea esculenta),* cassava *(Manihot esculenta),* potato *(Solanum tuberosum),* yam*(Dioscoreapolystachya),* groundnut *(Arachis hypogaea),* cranes peanut *(Arachis duranensis),* soybean *(Glycine max),* wild soybean *(Glycine soja),* adzuki bean *(Vigna angularis),* red bean *(Vigna umbellata),* lentil bean *(Lens culinaris),* mung bean *(Vigna radiata),* cowpea *(Vigna unguiculata),* kidney bean *(Phaseolus vulgaris),* broad bean *(Vicia faba),* pea *(Pisum sativum),* jack bean *(Canavalia gladiata),*pigeonpea *(Cajanus cajan),* white lupine *(Lupinus albus),* lather bean*(Mucuna pruriens),* narrow-leafed lupine *(Lupinus angustifolius),* hairy vine bean *(Calopogoniummucunoides),* chickpea *(Cicer arietinum),* sesame *(Sesamum indicum),* flax *(Linumusitatissimum),* rapeseed *(Brassica napus),* sunflower *(Helianthus annuus),* castor bean *(Ricinus communis),* red flower *(Carthamus tinctorius),* beet *(Beta vulgaris),* sugarcane *(Saccharum officinarum),* cotton *(Gossypium spp), marijuana(Cannabis sativa),* jiangnan rolling cypress *(Selaginella moellendorffii),* lemon eucalyptus *(Corymbia citriodora),* water green tree *(Tetracentronsinense),* mesquite tree *(Prosopis alba),* taxus chinensis *(Taxus chinensis),* olive *(Olea europaea),* African acacia*(Abrusprecatorius),* switchgrass *(Panicum virgatum),* red-vein maiguo *(Rhamnellarubrinervis),* American hickory *(Carya illinoinensis),* tea *(Camellia sinensis),* tobacco *(Nicotiana tabacum),* elephant grass *(Pennisetum purpureum),* hard-straight ryegrass *(Lolium rigidum),* nandi *(Miscanthuslutarioriparius),* canweed *(Setariaviridis),* cockweed *(Pennisetum alopecuroides),* Sudan grass *(Sorghum sudanense),* Tang pine grass *(Thalictrum thalictroides),* curved leaf thrush *(Eragrostiscurvula),* Brachypodium *(Brachypodiumdistachyon),* alfalfa *(LotuscorniculatusL),* Arabidopsis *(Arabidopsis thaliana),*Physcomitrella*(Physcomitrium patens),* hornmoss *(Ceratodonpurpureus),* cabbage *(Brassica oleracea),* mustard *(Brassica juncea),* Chinesegreen cabbage *(Brassica rapa),* cauliflower *(Brassica oleracea),* lettuce *(Lactuca sativa),* spinach *(Spinacia oleracea), radish(Raphanus sativus),* Chinesecabbage *(Brassica rapa),* zucchini *(Cucurbita pepo),* hemsley *(Apium graveolens),* leek *(Allium tuberosum),* carrot *(Daucus carota),* eggplant*(Solanum melongena),* tomato *(Lycopersicon esculentum),* cucumber *(Cucumis sativus),* wax gourd *(Benincasahispida),* pumpkin *(Cucurbita moschata),* loofah *(Luffa cylindrica),* vegetable melon *(Cucumis melo),* lotus vegetables *(Potentilla anserina),*day lily *(Hemerocallis citrina),* stem lettuce *(Lactuca sativa),* asparagus *(Asparagus officinalis),* chili pepper *(Capsicum annuum),* garlic *(Allium sativum),* spring onion *(Allium fistulosum),* Jerusalem *artichoke(Helianthus tuberosus),* Coriander *(Coriandrum sativum),* watermelon *(Citrullus lanatus),* pear *(Pyrus spp),* peach *(Prunus persica),* apricot *(Armeniaca vulgaris),* lee *(Prunus salicina),* date *(ZiZiphus jujuba),*green plum *(Vaticamangachapoi),* apple *(Malus pumila),* sand fruit *(Malus asiatica),* cherry *(Cerasus spp),* walnut *(Juglans regia),* strawberry *(Fragaria ananassa),* grape *(Vitis vinifera), pineapple* pear *(Ananas comosus),* coptis chinensis *(Coptis chinensis),* Chinese milk vetch *(Astragalus sinicus),* ginseng *(Panax ginseng),* Angelica sinensis *(Angelica sinensis),* honeysuckle *(Lonicera japonica),* Ai *(Artemisia argyi),* peppermint *(Mentha canadensis),* orchid *(Cymbidium ssp),* lily *(Lilium brownii),* tulip *(Tulipa gesneriana).*

7. A method of inducing plant haploids using a gene according to claim 2 to 4, comprising the steps of including the gene to obtain a positive transgene plant controlled by the promoter for specific expression in egg cells of the gene and, preferably, the plant including a monocot and a dicotyledon; preferred of all, the plant was selected from rice *(Oryza sativa),* wart-grain rice *(Oryza meyeriana varY,* maize*(Zea mays),* wheat *(Triticum aestivum),* durum wheat *(Triticum turgidum subsp),* wild emmer *wheat(Triticum dicoccoides),* urartu wheat *(Triticum urartu),* barley *(Hordeum vulgare),* section wheat *(Aegilops tauschii),* rye *(Secale cereale),* oats *(Avena sativa),* buckwheat *(Fagopyrum esculentum),* highland barley *(Hordeum vulgare),* semen coicis *(Coixlacryma-jobi)*, foxtail millet*(Setariaitalica),* fonio millet *(Digitariaexilis),* sorghum *(Sorghum bicolor),* proso *millet(Panicum miliaceum),* wild rice *(Zizania latifolia),* biotic wild rice *(Zizania palustris), coracana(Eleusine coracana),* sweet potato *(Dioscorea esculenta),* cassava *(Manihot esculenta),* potato *(Solanum tuberosum),* yam*(Dioscoreapolystachya),* groundnut *(Arachis hypogaea),* cranes peanut *(Arachis duranensis),* soybean *(Glycine max),* wild soybean *(Glycine soja),* adzuki bean *(Vigna angularis),* red bean *(Vigna umbellata),* lentil bean *(Lens culinaris),* mung bean *(Vigna radiata),* cowpea *(Vigna unguiculata),* kidney bean *(Phaseolus vulgaris),* broad bean *(Vicia faba),* pea *(Pisum sativum),* jack bean *(Canavalia gladiata),*pigeonpea *(Cajanus cajan),* white lupine *(Lupinus albus),* lather bean*(Mucuna pruriens),* narrow-leafed lupine *(Lupinus angustifolius),* hairy vine bean *(Calopogoniummucunoides),* chickpea *(Cicer arietinum),* sesame *(Sesamum indicum),* flax *(Linumusitatissimum),* rapeseed *(Brassica napus),* sunflower *(Helianthus annuus),* castor bean *(Ricinus communis),* red flower *(Carthamus tinctorius),* beet *(Beta vulgaris),* sugarcane *(Saccharum officinarum),* cotton *(Gossypium spp), marijuana(Cannabis sativa),* jiangnan rolling cypress *(Selaginella moellendorffii),* lemon eucalyptus *(Corymbia citriodora),* water green tree *(Tetracentronsinense),* mesquite tree *(Prosopis alba),* taxus chinensis *(Taxus chinensis),* olive *(Olea europaea),* African acacia*(Abrusprecatorius),* switchgrass *(Panicum virgatum),* red-vein maiguo *(Rhamnellarubrinervis),* American hickory *(Carya illinoinensis),* tea *(Camellia sinensis),* tobacco *(Nicotiana tabacum),* elephant grass *(Pennisetum purpureum),* hard-straight ryegrass *(Lolium rigidum),* nandi *(Miscanthuslutarioriparius),* canweed *(Setariaviridis),* cockweed *(Pennisetum alopecuroides),* Sudan grass *(Sorghum sudanense),* Tang pine grass *(Thalictrum thalictroides),* curved leaf thrush *(Eragrostiscurvula),* Brachypodium *(Brachypodiumdistachyon),* alfalfa*(LotuscorniculatusL),* Arabidopsis *(Arabidopsis thaliana),*Physcomitrella*(Physcomitrium patens),* hornmoss *(Ceratodonpurpureus),* cabbage *(Brassica oleracea),* mustard *(Brassica juncea),* Chinesegreen cabbage *(Brassica rapa),* cauliflower *(Brassica oleracea),* lettuce *(Lactuca sativa),* spinach *(Spinacia oleracea), radish(Raphanus sativus),* Chinesecabbage *(Brassica rapa),* zucchini *(Cucurbita pepo),* hemsley *(Apium graveolens),* leek *(Allium tuberosum),* carrot *(Daucus carota),* eggplant*(Solanum melongena),* tomato *(Lycopersicon esculentum),* cucumber *(Cucumis sativus),* wax gourd *(Benincasahispida),* pumpkin *(Cucurbita moschata),* loofah *(Luffa cylindrica),* vegetable melon *(Cucumis melo),* lotus vegetables *(Potentilla anserina),*day lily *(Hemerocallis citrina),* stem lettuce *(Lactuca sativa),* asparagus *(Asparagus officinalis),* chili pepper *(Capsicum annuum),* garlic *(Allium sativum),* spring onion *(Allium fistulosum),* Jerusalem artichoke *(Helianthus tuberosus),* Coriander *(Coriandrum sativum),* watermelon *(Citrullus lanatus),* pear *(Pyrus spp),* peach *(Prunus persica),* apricot *(Armeniaca vulgaris),* lee *(Prunus salicina),* date *(Ziziphus jujuba),*green plum *(Vaticamangachapoi),* apple *(Malus pumila),* sand fruit *(Malus asiatica),* cherry *(Cerasus spp),* walnut *(Juglans regia),* strawberry *(Fragaria ananassa),* grape *(Vitis vinifera), pineapple* pear *(Ananas comosus),* coptis chinensis *(Coptis chinensis),* Chinese milk vetch *(Astragalus sinicus),* ginseng *(Panax ginseng),* Angelica sinensis *(Angelica sinensis),* honeysuckle *(Lonicera japonica),* Ai *(Artemisia argyi),* peppermint *(Mentha canadensis),* orchid *(Cymbidium ssp),* lily *(Lilium brownii),* tulip *(Tulipa gesneriana).*

8. The method of claim 7, wherein the promoter for egg specific expression is a promoter of *AtDD45* egg specific expression; preferably with the nucleotide sequence shown in SEQ ID NO: 7; the import is an Agrobacterium-mediated or gene gun or gene editing method.

9. The method of claim 7 or 8, comprising the step of taking transgenic positive material to obtain haploid material.

10. The method of claim 9, further comprising combining haploid inductive material to obtain apomixis material with a *MiMe* system.
